# EUROPEAN PATENT SPECIFICATION

(11) **EP 3 976 054 B1**
(45) Date of publication and mention of the grant of the patent: **07.01.2026**
(21) Application number: 20814645.6
(22) Date of filing: 22.05.2020
(51) Int. Cl.: A61K 31/7068, A61K 38/19, A61K 45/06, A61P 35/00

(54) **TIMED ADMINISTRATION OF DECITABINE AND 5-AZACYTIDINE FOR CANCER TREATMENT**
ZEITLICH FESTGELEGTE VERABREICHUNG VON DECITABIN UND 5-AZACYTIDIN ZUR KREBSBEHANDLUNG
ADMINISTRATION SYNCHRONISÉE DE DÉCITABINE ET DE 5-AZACYTIDINE POUR LE TRAITEMENT DU CANCER

(30) Priority: 24.05.2019 US 201962852807 P
(43) Date of publication of application: 06.04.2022
(73) Proprietor: The Cleveland Clinic Foundation, Cleveland, OH 44195 (US)
(72) Inventor: SAUNTHARARAJAH, Yogen, Cleveland, Ohio 44195 (US); VELCHETI, Vamsidhar, Cleveland, Ohio 44195 (US); KANG, Kai, Cleveland, Ohio 44195 (US); GU, Xiaorong, Cleveland, Ohio 44195 (US); TOHME, Rita, Cleveland, Ohio 44195 (US)
(74) Representative: Glawe, Delfs, Moll
(86) International application number: PCT/US2020/034321
(87) International publication number: WO 2020/242983

(56) References cited:
- WO-A1-2017/096357
- WO-A1-2017/167989
- US-B2- 9 265 785
- US-B2- 9 895 391
- KANG KAI ET AL: "Tetrahydrouridine/decitabine/5-azacytidine for non-cytotoxic epigenetic-immunotherapy of NSCLC in vivo; Abstract e24134", vol. 36, no. 15_suppl, 1 June 2018 (2018-06-01), XP093047137, Retrieved from the Internet <URL:https://ascopubs.org/doi/abs/10.1200/JCO.2018.36.15_suppl.e24134>
- EBRAHEM QUTEBA ET AL: "Mechanisms of Resistance to 5-Azacytidine/Decitabine in MDS-AML and Pre-Clinical In Vivo Proof of Principle of Rational Solutions to Extend Response", BLOOD, AMERICAN SOCIETY OF HEMATOLOGY, US, vol. 126, no. 23, 3 December 2015 (2015-12-03), pages 678, XP086645939, ISSN: 0006-4971, DOI: 10.1182/BLOOD.V126.23.678.678
- SAUNTHARARAJAH YOGEN ET AL: "Evaluation of noncytotoxic DNMT1-depleting therapy in patients with myelodysplastic syndromes", THE JOURNAL OF CLINICAL INVESTIGATION, vol. 125, no. 3, 26 January 2015 (2015-01-26), GB, pages 1043 - 1055, XP093047361, ISSN: 0021-9738, DOI: 10.1172/JCI78789
- OSCAR ALCAZAR ET AL: "Epigenetic regulation by decitabine of melanoma differentiation in vitro and in vivo", INTERNATIONAL JOURNAL OF CANCER, JOHN WILEY & SONS, INC, US, vol. 131, no. 1, 6 September 2011 (2011-09-06), pages 18 - 29, XP071286556, ISSN: 0020-7136, DOI: 10.1002/IJC.26320
- "Granulocyte colony-stimulating factor", WIKIPEDIA, 7 April 2019 (2019-04-07), XP055763758, Retrieved from the Internet <URL:https://en.wikipedia.org/w/index.php?title=Granulocyte_colony-stimulating_factor&oldid=891379136>

## Description

The present application claims priority to U.S. Provisional application 62/852,807, filed May 24, 2019.

### FIELD

Provided herein are compositions, systems, kits, and methods for treating a patient with cancer by alternate administration of decitabine and 5-azacytidine, in particular as per the treatment regime as defined in claim 1. Such administration is combined with an inhibitor of the enzyme cytidine deaminase (e.g., tetrahydrouridine) that otherwise rapidly catabolizes decitabine and 5-azacytidine. The time between cycles of decitabine and 5-azacytidine administration is about three to four days as defined in claim 1.

### BACKGROUND

Lung cancer is the leading cause of cancer mortality world-wide, claiming ~1.7 million lives per year, more than liver and colorectal cancers combined. A landmark recent advance was approval of immune checkpoint blockade (ICB) using anti-PD-1/PD-L1 monoclonal antibodies to treat metastatic lung cancer. Unfortunately, only ~20% of patients treated this way have durable benefits (1). New and rational complementary therapies are thus needed.

Lung cancers contain a high mutational burden that should require broader dependence on immune checkpoints for immune evasion than response rates to ICB thus far indicate (2). To be recognized by T-cells, however, antigenic peptides must be processed by proteasomes and chaperoned to the cancer cell surface for presentation by major histocompatibility complex (MHC) molecules. Any step in this antigen presentation process can be downregulated by repressive epigenetic changes, e.g., DNA methylation by DNA methyltransferase 1 (DNMT1) (reviewed in (3-5)). Accordingly, in several pre-clinical cancer models, DNMT1-depletion by the pyrimidine nucleoside analogs decitabine or 5-azacytidine increased expression of MHC molecules and associated immune co-stimulatory molecules, promoted tumor infiltration by IFN-γ producing and cytotoxic T-cells, and augmented anti-tumor effects of ICB (3-5). Transcription repression by DNMT1 and DNA methylation moreover silences other tumor suppressor genes in cancer cells, e.g., epithelial-differentiation genes which would otherwise antagonize the master regulator of cell growth and division MYC and terminate cancer cell self-renewal (reviewed in (6)). That is, DNMT1-depletion has been shown to not just augment immune-recognition of tumors, and hence enhance efficacy of immune checkpoint inhibitors, but also directly cytoreduces cancers, by a non-cytotoxic (non-apoptosis based) pathway that operates even in p53-null cancers refractory to apoptosis-based, and immune-disrupting, chemotherapy and radiation (6).

Overall, however, clinical results with decitabine or 5-azacytidine to treat solid tumor malignancies, alone or in combination with ICB, have not been as impressive as predicted by pre-clinical data (reviewed in (7)). One reason became evident in a clinical trial of intravenously-infused decitabine in patients with thoracic malignancies. Despite sustaining decitabine plasma concentrations of >40nM for ~72 hours, enough to cause grade 3/4 myelosuppression, molecular pharmacodynamic effects of DNA hypomethylation in lung cancer tissues were evident in <25% of patients (8). This indicated that levels of active drug in lung cancer tissues *in vivo* were minimal despite drug accumulation to excessive, cytotoxic levels in bone marrow. A reason for such uneven tissue effects was suggested by subsequent experiments showing that the catabolic enzyme cytidine deaminase (CDA), that deaminates decitabine and 5-azacytidine into non-DNMT1-depleting metabolites within minutes, is expressed unevenly across tissues (9-12), being highly expressed in many solid tissues (e.g., liver) but less so in others (e.g., bone marrow). Accordingly, decitabine was ineffective in a pre-clinical model of cancer localized to the liver, but co-administration of lower doses of decitabine with an inhibitor of CDA, tetrahydrouridine (THU), eliminated cancer cells in this CDA-rich organ sanctuary, importantly, without bone marrow cytotoxicity (9). Also noteworthy is that CDA levels are in general much higher in humans than in mice (13), a feature possibly contributing to general discrepancies between murine and human studies.

The prior art documents WO 2017/096357 A1, Kang et al., "Tetrahydrouridine/ decitabine/5-azacytidine for non-cytotoxic epigenetic-immunotherapy of NSCLC in vivo, Abstract e24134" Journal of Clinical Oncology, vol. 36, no. 15_suppl, 1 June 2018, and Ebrahem et al., "Mechanisms of Resistance to 5-Azacytidine/Decitabine in MDS-AML and Pre-Clinical In Vivo Proof of Principle of Rational Solutions to Extend Response", Blood, American Society of Hematology, vol. 126, no. 23, 3 December 2015, page 678 can be considered as technological background for the present invention as defined in the claims. None of said prior art documents disclose a dosage regimen as defined in the claims that enables practical translation into safe, efficacious and sustainable treatment of a patient with cancer.

### SUMMARY

The invention is set out in the appended claims. The embodiments of the description which do not fall within the scope of said claims are provided for illustrative purposes only and do not form part of the present invention. Provided herein are compositions, systems, kits, and methods for treating a patient with cancer by alternate administration of decitabine and 5-azacytidine, in particular as per the treatment regime as defined in claim 1. Such administration is combined with an inhibitor of the enzyme cytidine deaminase (e.g., tetrahydrouridine) that otherwise rapidly catabolizes decitabine and 5-azacytidine. The time between cycles of decitabine and 5-azacytidine administration is about three to four days as defined in claim 1. The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

Not comprised by the claims, but considered to be useful for aspects of the claimed invention, are methods of treating a human subject with cancer comprising, or consisting essentially of: a) administering a cytidine deaminase inhibitor to a human subject at a first time, wherein the human subject has cancer; b) administering decitabine to the subject at a second time, wherein the second time is between 0.5 and 7 hours after the first time; c) administering a cytidine deaminase inhibitor to the subject at a third time, wherein the third time is between 60 and 105 hours, or 106-240 hours, after the first time; and d) administering 5-azacytidine to the subject at a fourth time, wherein the fourth time is: i) 0.5 and 7 hours after the third time, and ii) between 60 and 105 hours, or 106-240 hours, after the second time. The methods may further comprise: e) repeating steps a) - d) at least once, wherein the administering in step a) is conducted between 60 and 105 hours, or 106-240 hours, after the fourth time.

Not comprised by the claims, but considered to be useful for aspects of the claimed invention, are methods of treating a human subject with cancer comprising, or consisting essentially of: a) administering 5-15 mg/kg of a cytidine deaminase inhibitor to a human subject at a first time, wherein the human subject has cancer; b) administering 0.07-0.4 mg/kg of decitabine to the subject at a second time, wherein the second time is between 0.5 and 7 hours after the first time; c) administering 5-15 mg/kg of a cytidine deaminase inhibitor to the subject at a third time, wherein the third time is between 60 and 105 hours, or 106-240 hours, after the first time; and d) administering 0.5-4 mg/kg of 5-azacytidine to the subject at a fourth time, wherein the fourth time is: i) 0.5 and 7 hours after the third time, and ii) between 60 and 105 hours, or 106-240 hours, after the second time. The methods may further comprise repeating steps a) - d) at least once, wherein the administering in step a) is conducted between 60 and 105 hours, or 106-240 hours, after the fourth time.

The subject may not receive any decitabine or 5-azacytidine except at the times specified in steps b) and d) respectively. The repeating steps a) - d) at least once may comprise repeating steps a) - d) at last five times (e.g., 5, 6, 7, 8, 9, 10, or 11 times). The repeating steps a) - d) at least once may comprise repeating steps a) - d) at last twelve times (e.g., 12, 13, 14, 15, 16, 17, 18 ... 25 ... or 50 times).

The cancer may be selected from the group consisting of: pancreatic cancer, breast cancer, myeloid cancers, lymphoid cancers (e.g., T-cell lymphoid cancers), small cell lung cancer, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, and retinoblastoma.

The administering 5-15 mg/kg of the cytidine deaminase inhibitor in steps a) and c) may comprise administering 9-11 mg/kg of the cytidine deaminase inhibitor. The administering 0.07-0.4 mg/kg of decitabine may comprise administering 0.1-0.3 mg/kg of the decitabine. The administering 0.07-0.4 mg/kg of decitabine may comprise administering 0.15-0.17 mg/kg of the decitabine. The administering 0.5-4 mg/kg of 5-azacytidine may comprise administering 1-3 mg/kg of the 5-azacytidine. The administering 0.5-4 mg/kg of 5-azacytidine may comprise administering 1.5-1.7 mg/kg of the 5-azacytidine.

The between 0.5 and 7 hours in step b) may be between 1 and 5 hours (e.g., 1, 2, 3, 4, or 5 hours). The between 0.5 and 7 hours in step d) may be between 1 and 5 hours (e.g., 1, 2, 3, 4, or 5 hours). The between 60 and 105 hours, or 106-240 hours, in step d) may be between 70 and 98 hours or between 72 and 96 hours (e.g., 72 ... 80 ... 88 ... 96 hours) or between 150 and 175 hours (e.g., 150 ... 155 ... 170 ... 175). The between 60 and 105 hours, or 106-240 hours, in step c) may be between 65 and 100 hours (e.g., 65 ... 75 ... 85 ... 95 ... and 100 hours), or between 150 and 175 hours (e.g., 150 ... 155 ... 170 ... 175).

As defined in claim 7, administering the subject a composition comprising human granulocyte colony-stimulating factor (GCSF) may be further comprised. As defined in claim 8, testing a sample from the subject to determine absolute neutrophil count (ANC), wherein the subject's ANC is preferably below 1.5x10⁹, and administering the subject a composition comprising human granulocyte colony-stimulating factor (GCSF) may be further comprised. As defined in claim 9, the subject may not receive any cytidine deaminase inhibitor between said administration of decitabine and 5-azacytidine combined with said cytidine deaminase inhibitor as defined in claim 1. As defined in claim 10, the cytidine deaminase inhibitor may comprise tetrahydrouridine.

Not comprised by the claims, but considered to be useful for aspects of the claimed invention, are methods of treating a human subject with cancer comprising, or consisting essentially of: a) administering decitabine to the subject on day one; b) administering decitabine to the subject on day two, wherein day two is the day immediately after the day one; c) administering decitabine to the subject on day eight, wherein the day eight is seven days after the day one, and wherein the subject does not receive any decitabine between the administering in step b) and the administering in step c); and d) administering decitabine to the subject on day nine, wherein the day nine is eight days after the day one.

The methods may further comprise: repeating steps c) and d) at least once, wherein the subject does not receive any decitabine expect during the repeated administration of steps c) and d). Steps c) and d) may be repeated at least twice (e.g., 2 times, 3 times, 4 times, 5 times, 6 times, 7 times, 8 times, 9 time, or 10 times). The methods may further comprise administering a cytidine deaminase inhibitor to the subject on the same days as when the decitabine is administered. The cytidine deaminase inhibitor may be administered at a dosage of 5-15 mg/kg, as defined in claim 5. Each administration of the decitabine may be administered at dosage of 0.07-0.4 mg/kg, as defined in claim 6.

### DESCRIPTION OF THE FIGURES

The patent or application file contains at least one drawing executed in color. Copies of this patent or patent application publication with color drawings will be provided by the Office upon request and payment of the necessary fee.

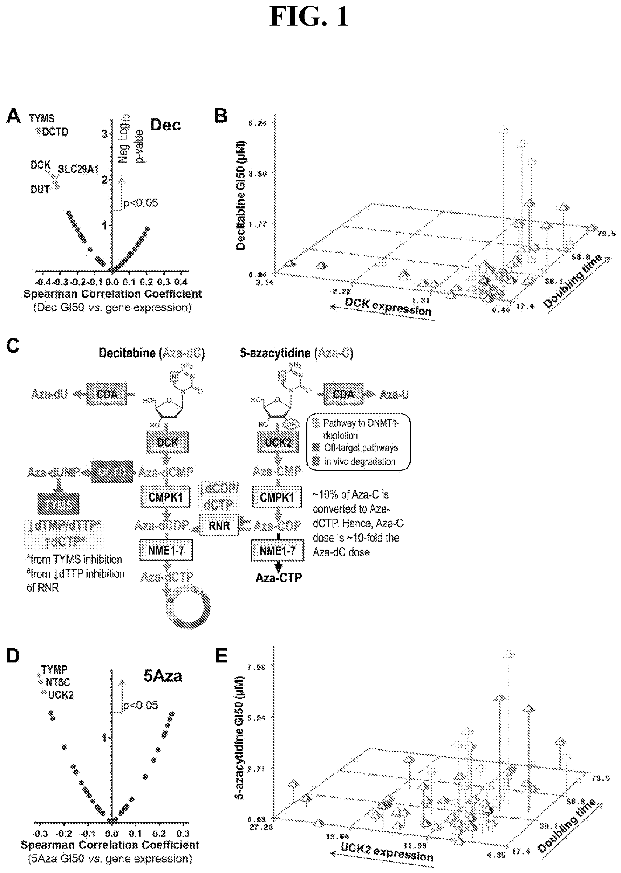

Figure 1. Expression levels of key pyrimidine metabolism enzymes determine sensitivity of diverse cancer cell lines to decitabine (Dec) and 5-azacytidine (5Aza). A) Dec sensitivity correlates with DCK expression. Unbiased correlation of expression levels of pyrimidine metabolism enzymes and transporters (n=45) vs decitabine sensitivity (concentration of drug producing 50% growth inhibition at day 6 [GI50]) in the NCI60 panel of cancer cell lines (n=60), public GI50 data, National Cancer Institute. Microarray gene expression data from GSE5846(47). Spearman correlation coefficients/p-values by SASv9. p-values <0.05 highlighted in green. B) Three-way plot of Dec GI50 *vs* DCK expression and doubling time. Cell line histologies: leukemia=blue, non-small cell lung=red, colon=green, central nervous system=gold, melanoma=grey, ovarian=pink, renal=light blue, prostate=black, breast=violet. C) Dec and 5Aza are converted into the DNMT1-depleting nucleotide by separate channels (green). Also shown are off-target effects (red) that disrupt nucleotide balances (yellow background).D) 5Aza sensitivity correlates with UCK2 expression. E) Three-way plot of 5Aza GI50 *vs* UCK2 expression and doubling time.

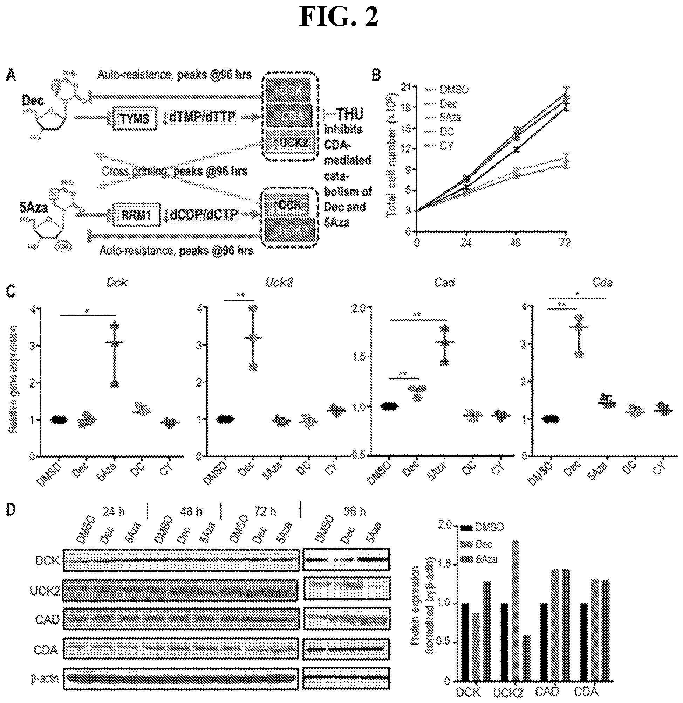

Figure 2. The pyrimidine metabolism network reacts to Dec and 5Aza perturbation with adaptive shifts in enzyme expression that decrease nucleoside uptake. F1339 p53-null cancer cells were seeded at D0 and treated with DMSO, deoxycytidine (DC, 0.5 µM), cytidine (CY, 5 µM), Dec (0.5 µM) and 5Aza (5 µM). A) Dec has been shown to inhibit TYMS (17, 48) and 5Aza to inhibit ribonucleotide reductase (RRM1) (21), disrupting cellular nucleotide balances in specific ways. The enzyme expression shifts dampen uptake of the instigating drug (auto-resistance) but prime for uptake of the sister drug (cross-priming). CDA, that is upregulated by Dec, inactivates both Dec and 5Aza, and can be inhibited by tetrahydrouridine (THU). B) Dec and 5Aza, but not the natural nucleosides, slowed cell growth. Cell counts by automated counter, mean ± SD. C) Dec increased UCK2 and decreased DCK, while 5Aza did the opposite. Gene expression by Q-PCR, median±interquartile range (IQR) (3 biological replicates), **p <* 0.05, ***p <* 0.01. 2-sided Mann-Whitney test. D) Protein expression tracked the mRNA expression changes. Western blot.

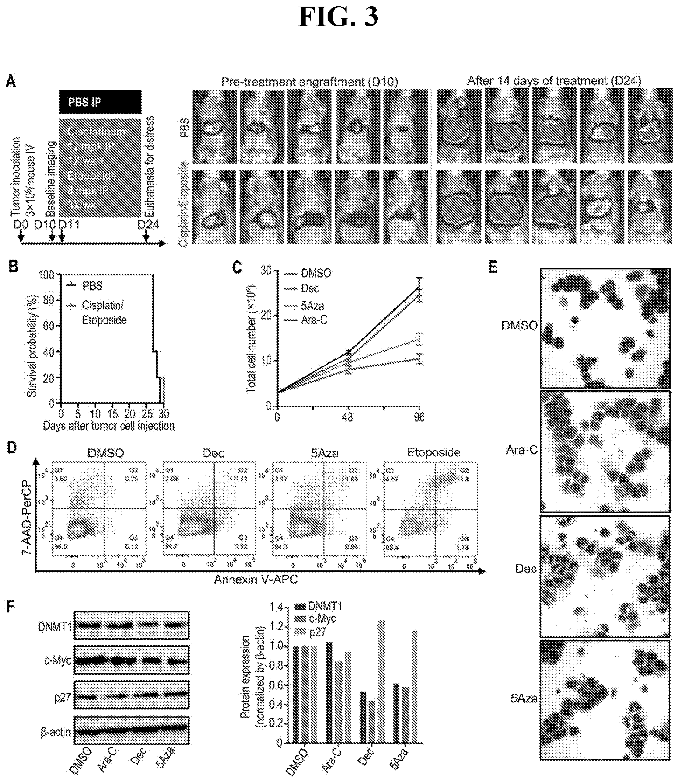

Figure 3. Non-cytotoxic concentrations of Dec and 5Aza suppress chemorefractory p53-null SCLC cells. A) High doses of etoposide and cisplatin were ineffective in vivo. B6/129 F1 mice were inoculated via tail vein with F1339-luc SCLC cells (0.3×10⁶cells/mouse). After documentation of lung invasion by live-imaging, mice were distributed to PBS or combination cisplatin 12 mg/kg intraperitoneal (IP) 1X/week + etoposide 8 mg/kg IP 3X/week (n = 5/group). B) No benefit of cisplatin + etoposide. Mice were euthanized for signs of distress. C) Dec and 5Aza, but not cyarabine at a concentration equimolar to the Dec, was anti-proliferative to p53-null SCLC cells. Dec and cytarabine 0.5 µM, 5Aza 5 µM on Day 1. Cell counts by automated counter. Mean±SD. D) The anti-proliferative action of Dec and 5Aza was not via early apoptosis. Cell membrane Annexin-V staining measured by flow-cytometry on Day 2. Positive control used etoposide at 400 µg/ml. E) Dec and 5Aza treatment, but not cytarabine, induced morphologic changes of differentiation (decreased nuclear cytoplasmic ratio, increased cytoplasmic complexity). Giemsa-stained cytospins Day 5 (drug added Day 1). F) Dec and 5Aza, but not cytarabine, decreased Myc and increased p27/Cdkn1B. Western blot Day 5 (drug added Day 1). Graph shows results of densometric analysis.

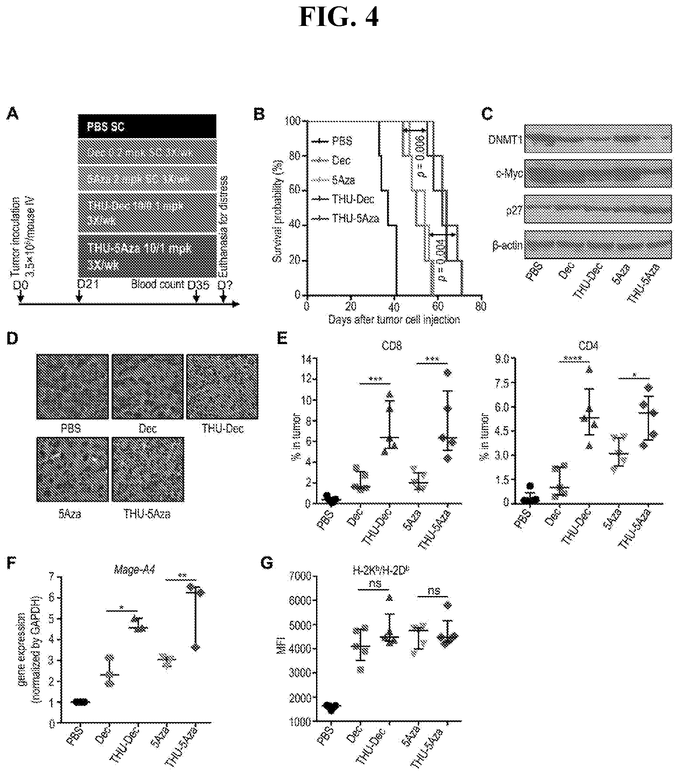

Figure 4: The addition of THU increased lung cancer cytoreduction and immune-priming over Dec or 5Aza alone. A) Experiment schema. C57/BL6 mice were inoculated via tail vein with LL3 lung cancer cells (0.35×10⁶cells/mouse. Mice were randomized to PBS, Dec (0.2 mg/kg sc 3×/wk), THU-Dec (10/0.1 mg/kg sc 3 ×/wk), 5Aza (2 mg/kg sc 3 ×/wk), or THU-5Aza (10/1 mg/kg sc 3×/wk)(n = 5/group). B) THU addition significantly increased time-to-distress over Dec or 5Aza alone. p-values Log Rank test. C) THU addition produced more DNMT1-depletion, MYC reduction and p27 upregulation over Dec or 5Aza alone. Western blots of pooled tumor tissue harvested at euthanasia. D) THU addition decreased nuclear:cytoplasmic ratio over Dec or 5Aza alone. H&E staining of tumor sections obtained at euthanasia. E) THU addition increased tumor infiltration by immune-effector cells over Dec or 5Aza alone. Measured by flow cytometry of homogenized tumor tissue. F) THU addition increased cancer testes expression over Dec or 5Aza alone. Measured by QRT-PCR. G) THU addition increased MHC class 2 expression over Dec or 5Aza alone. H-2K^{b}/H-2D^{b} expression in tumor tissue measured by flow cytometry of dissociated tumor tissue. Bar and whiskers = median ± IQR. **p <* 0.05; ***p <* 0.01; ****p <* 0.001; *****p <* 0.0001; ns: *p >* 0.05. 2-sided Mann-Whitney test.

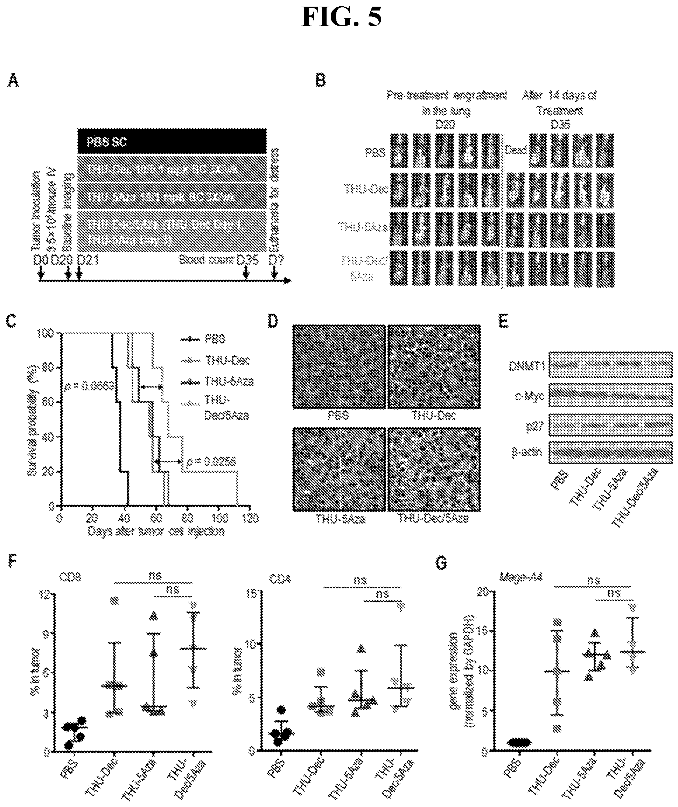

Figure 5: Alternating THU-Dec with THU-5Aza enhanced pharmacodynamic effect and cytoreduction over THU-Dec or THU-5Aza alone. A) Experiment schema. C57BL/6 mice were inoculated via tail vein with LL3-luc lung cancer cells (0.35×10⁶cells/mouse. After documentation of lung invasion by live-imaging, mice were distributed to PBS, THU-Dec (10/0.1 mg/kg sc 3×/wk), THU-5Aza (10/1 mg/kg sc 3×/wk), and THU-Dec/THU-5Aza (THU-Dec 10/0.1 mg/kg sc 2×/wk then THU-5Aza 10/1 mg/kg sc on the third day). B) Tumor burden before and during treatment with the different regimens. Live-imaging. C) The alternating regimen significantly increased time-to-distress over THU-Dec or THU-5Aza alone. p-values Log Rank test. D) The alternating regimen decreased nuclear:cytoplasmic ratio over THU-Dec or THU-5Aza alone. H&E staining of tumor sections obtained at euthanasia. E) The alternating regimen produced more DNMT1-depletion, Myc reduction and p27 upregulation over THU-Dec or THU-5Aza alone. Western blots of pooled tumor tissue harvested at euthanasia. F) Effects of the different regimens on tumor infiltration by immune-effector cells. Measured by flow cytometry of dissociated tumor tissue. G) Effects of the different regimens on tumor cancer testis antigen expression. Bar and whiskers = median ± IQR. ns: *p >* 0.05. 2-sided Mann-Whitney test.

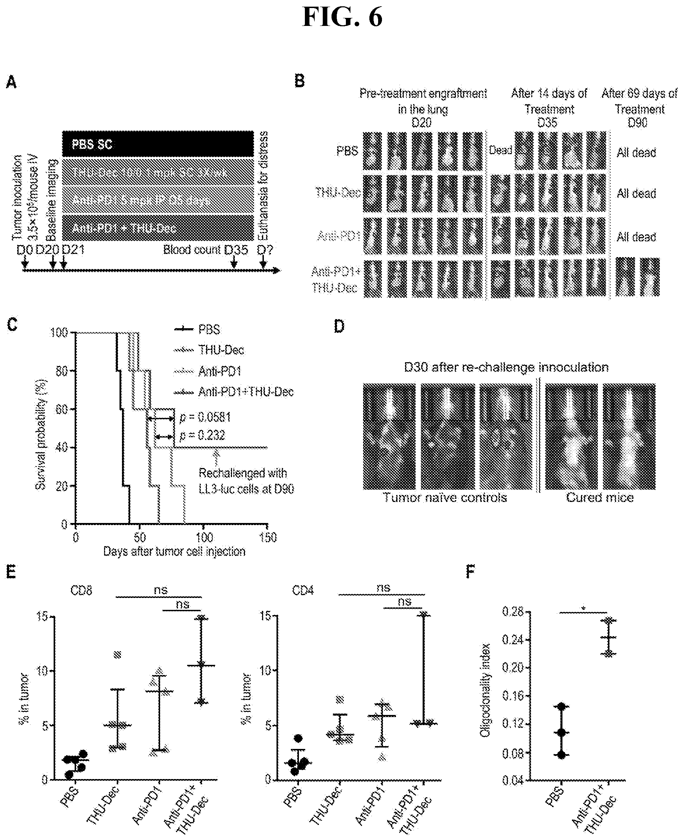

Figure 6: THU-Dec enhanced the immuno-therapeutic effects of anti-Pd1. C57BL/6 mice were inoculated via tail vein with LL3-luc lung cancer cells (0.35×10⁶cells/mouse). After documentation of lung invasion by live-imaging, mice (n = 5/group) were randomized to treatment with PBS, THU-Dec (10/0.1 mg/kg sc 3×/wk), anti-PD1 (5 mg/kg ip q5d) or anti-PD1+THU-Dec combination. A) Experiment schema. B) Tumor burden before and during treatment with the different regimens. Live-imaging. C) Combination anti-PD1+THU-Dec produced the greatest increases in time-to-distress over the other treatments, including two apparent cures. p-values Log Rank test. D) Cured mice and tumor-naïve control mice were challenged with LL3-luc cells. Tumor engraftment measured by live imaging. E) Effects of the different regimens on tumor infiltration by immune-effector cells. Measured by flow cytometry of dissociated tumor tissue. F) PB T-cell oligoclonality was significantly increased in mice cured by the combination treatment vs vehicle-treated controls. Oligoclonality index: percentage of the overall T-cell population represented by the 1000 most abundant T-cell clones, measured by T-cell receptor (TCR) RNA sequencing. Bar and whiskers = Median ± IQR; **p <* 0.05; ns: *p >* 0.05. 2-sided Mann-Whitney test.

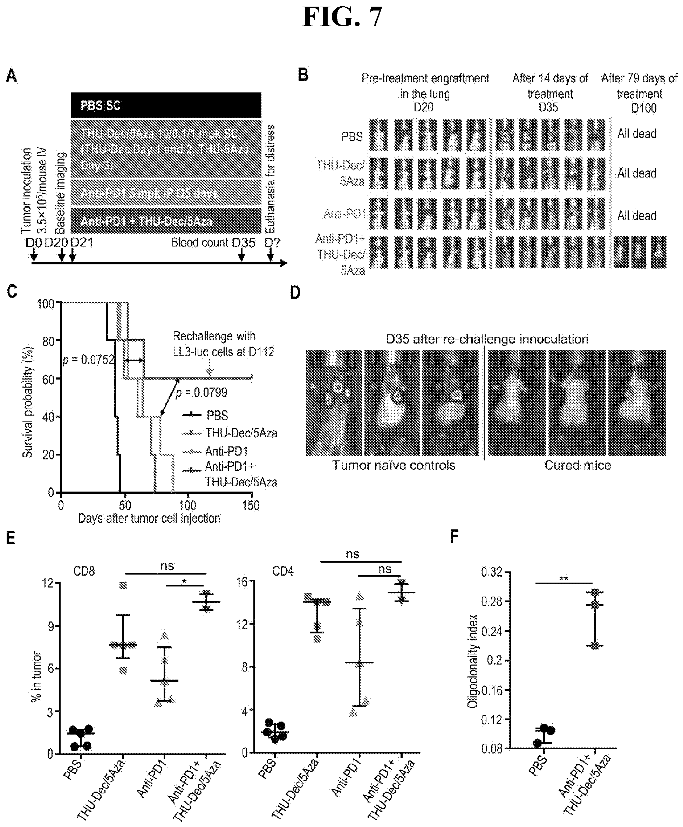

Figure 7: Alternating THU-Dec with THU-5Aza further enhanced the immuno-therapeutic effects of anti-PD1. C57BL/6 mice were inoculated via tail vein with LL3-luc lung cancer cells (0.35×10⁶cells/mouse). After documentation of lung invasion by live-imaging, mice (n = 5/group) were distributed to PBS, THU-Dec 10/0.1 SC 2×/wk followed by THU-5Aza 10/1 mg/kg SC 1×/wk), anti-PD1 (5 mg/kg IP q5d) or anti-PD1+THU-Dec/5Aza combination. A) Experiment schema. B) Tumor burden before and during treatment with the different regimens. Live-imaging. C) Combination anti-PD1+THU-Dec/5Aza produced the greatest increases in time-to-distress over the other treatments, including three apparent cures. p-values Log Rank test. D) Cured mice and tumor-naive control mice were challenged with LL3-luc cells. Tumor engraftment measured by live imaging. E) Effects of the different regimens on tumor infiltration by immune-effector cells. Measured by flow cytometry of homogenized tumor tissue. F) PB T-cell oligoclonality was significantly increased in mice cured by the combination treatment vs vehicle-treated controls. Oligoclonality index = percentage of the overall T-cell population represented by the 1000 most abundant T-cell clones, measured by T-cell receptor (TCR) RNA sequencing. Bar and whiskers = Median ± IQR; **p <* 0.05; ***p <* 0.01; ns: *p >* 0.05. 2-sided Mann-Whitney test.

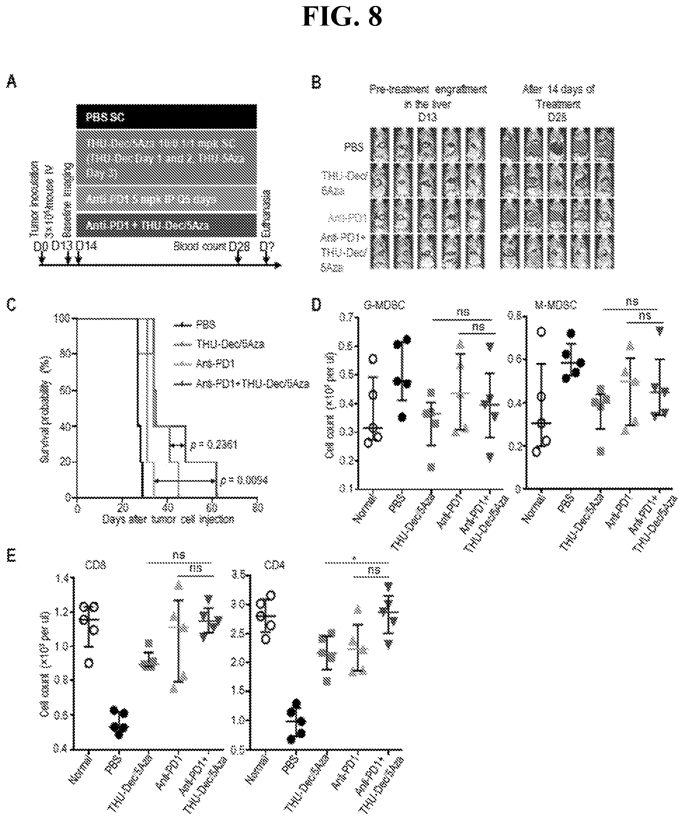

Figure 8. THU-Dec/5Aza alternating treatment and combination epigenetic-immunotherapy were of benefit in the SCLC model, but anti-PD1 alone was of minimal benefit. B6/129 F1 mice were inoculated via tail vein with F1339-luc SCLC cells (0.3×10⁶cells/mouse). After documentation of tumor invasion by live-imaging, mice (n = 5/group) were randomized to PBS, THU-Dec/5Aza, anti-PD1 or anti-PD1+THU-Dec/5Aza combination. A) Experiment schema. B) Tumor burden before and during treatment with the different regimens. Live-imaging. C) Combination anti-PD1+THU-Dec/5Aza produced the greatest increases in time-to-distress over the other treatments; anti-PD1 alone appeared to be of minimal or no benefit. p-values Log Rank test. D) Combination anti-PD1+THU-Dec/5Aza decreased PB granulocytic and monocytic myeloid-derived suppressor cells (G-MDSC and M-MDSC). E) Peripheral blood CD8⁺ and CD4⁺ T-cells were significantly and similarly increased. PB counts of the various cell types, measurements by flow cytometry. Bar and whiskers = Median±IQR. **p <* 0.05, ns: *p >* 0.05. 2-sided Mann-Whitney test.

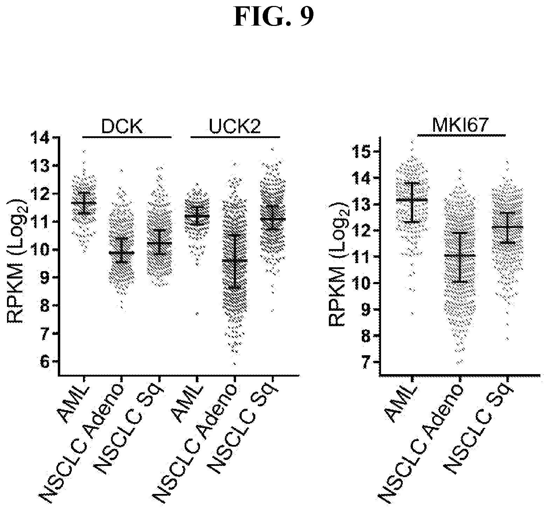

Figure 9. Myeloid malignancies (e.g., acute myeloid leukemia, AML), the only malignancies for which decitabine and 5-azacytidine are currently approved or routinely used as therapy, express up to several-fold higher levels of DCK and UCK2, and have a higher growth fraction (represented by MKI67 expression) than non-small cell lung cancer (NSCLC) (adeno = adenocarcinoma; squam = squamous carcinoma). Gene expression by RNA-sequencing by TCGA, AML n=173, NSCLC Adeno n=515, NSCLC squam n=498.

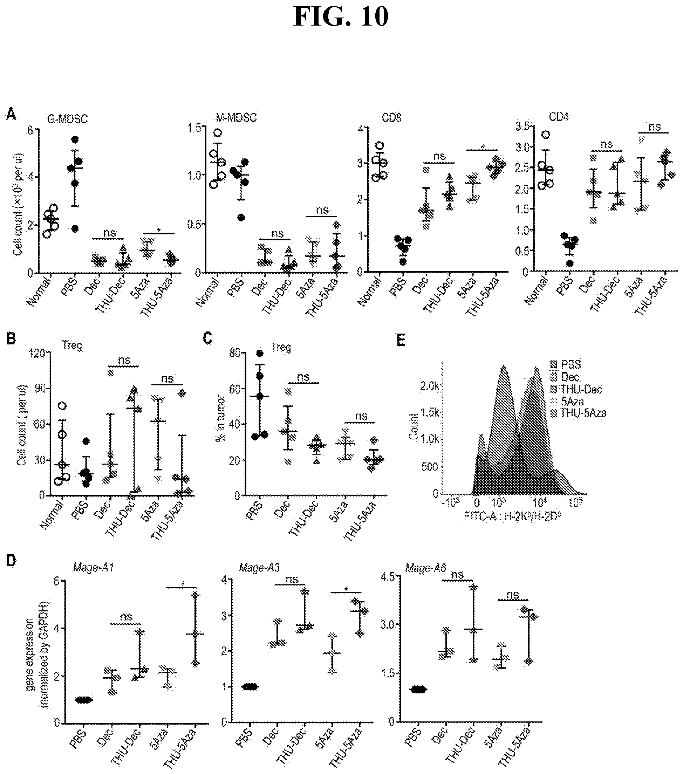

Figure 10. Effects of Dec or 5Aza vs tetrahydrouridine-Dec (THU-Dec) or THU-5Aza on peripheral blood (PB) immune-suppressor and immune-effector cell numbers after 14 days of treatment. C57BL/6 mice were inoculated via tail vein with LL3 lung cancer cells (0.35×10⁶cells/mouse). Mice were randomized to PBS, Dec (0.2 mg/kg sc 3×/wk), THU-Dec (10/0.1 mg/kg sc 3×/wk), 5Aza (2 mg/kg sc 3×/wk), or THU-5Aza (10/1 mg/kg sc 3×/wk)(n = 5/group). A) PB granulocytic and monocytic myeloid-derived suppressor cells (G-MDSC and M-MDSC) were significantly and similarly decreased (~4-fold) by the various treatments versus vehicle, while peripheral blood CD8⁺ and CD4⁺ T-cells were significantly and similarly increased. B) In PB, numbers of peripheral blood regulatory T cells (T_{reg}) were not significantly changed by the treatments vs vehicle. C) In tumor tissue, T_{reg} were similarly decreased by all the treatments. D) Suggesting a basis for increased T cell infiltration of tumor, expression of tumor-testes antigens (Mage-A1, Mage-A3, Mage-A6) was increased the most (up to 6-fold) by THU-Dec or THU-5Aza. E) The antigen presenting molecule MHC class I H-2K^{b}/H-2D^{b} was similarly increased by ~2-fold by all the treatments versus vehicle. Bar and whiskers = median ± IQR. **p* < 0.05; ns: *p >* 0.05. 2-sided Mann-Whitney test.

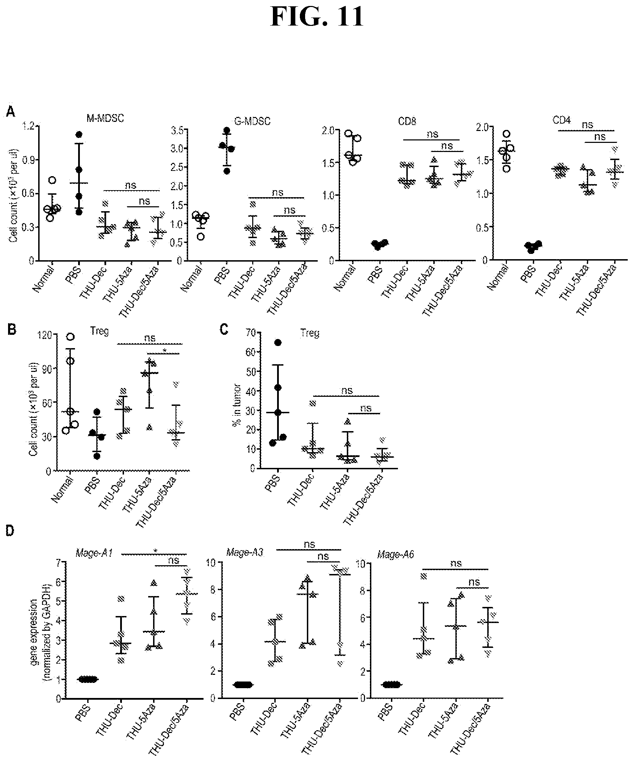

Figure 11. THU-Dec/THU-5Aza alternating treatment enhanced the effects over THU-Dec or THU-5Aza alone. C57BL/6 mice were inoculated via tail vein with LL3-luc lung cancer cells (0.35×10⁶cells/mouse). After documentation of lung invasion by live-imaging, mice (n = 5/group) were distributed to PBS, THU/decitabine (10/0.1 mg/kg sc 3×/wk), THU/5-azacytidine (10/1 mg/kg sc 3×/wk), THU/decitabine/5-azacytidine alternating (THU/decitabine 10/0.1 mg/kg sc 2×/wk followed with THU/5-azacytidine (10/1 mg/kg sc on the third day). A) Absolute numbers of peripheral blood G-MDSC and M-MDSC were significantly and similarly decreased for the different treatments versus vehicle, while peripheral blood CD8⁺ and CD4⁺ T-cells were significantly and similarly increased. B) In PB, numbers of peripheral blood regulatory T_{reg} were not significantly changed by the treatments vs vehicle. C) In tumor tissue, T_{reg} were similarly decreased by all the treatments vs vehicle. D) All three treatment regimens similarly increased expression of tumor-testes antigens (Mage-A1, Mage-A3, Mage-A6) vs vehicle. Bar and whiskers = median ± IQR. **p* < 0.05; ns: *p* > 0.05. 2-sided Mann-Whitney test.

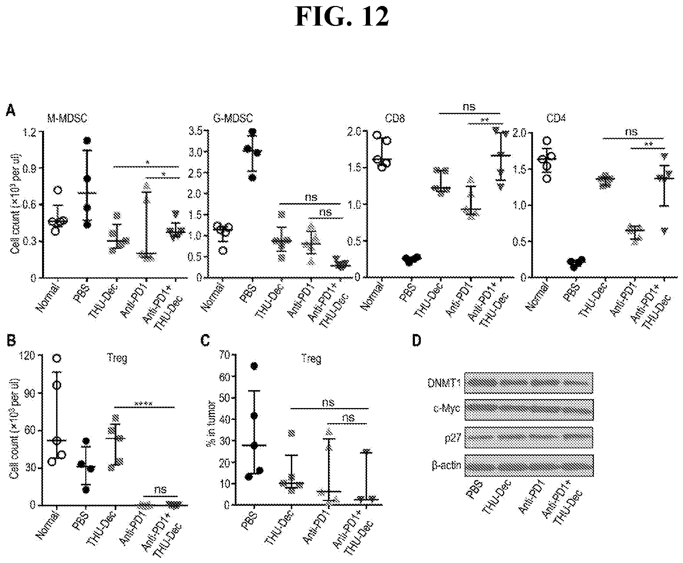

Figure 12. THU-Dec enhanced the immuno-therapeutic effects of anti-Pd1. C57BL/6 mice were inoculated via tail vein with LL3-luc lung cancer cells (0.35×10⁶cells/mouse. After documentation of lung invasion by live-imaging, mice (n = 5/group) were distributed to PBS, THU-Dec (10/0.1 mg/kg SC 3×/wk), anti-Pd1 (5 mg/kg IP q5d) or anti-Pd1+THU-Dec combination. A) Absolute numbers of peripheral blood G-MDSC and M-MDSC were significantly and similarly decreased by the different treatments vs vehicle, while PB CD8⁺ and CD4⁺ T-cells were significantly and similarly increased. B) PB T_{reg} were significantly decreased by anti-Pd1 and the anti-Pd1/THU-decitabine combination vs the other treatments. C) T_{reg} infiltration of tumor was similarly decreased by all treatments vs vehicle but not to a significant extent. D) Dnmt1-depletion, Myc-downregulation and p27/Cdkn1b upregulation was greatest with the combination treatment. Western blot of pooled tumor tissue from multiple mice. Bar and whiskers = median ± IQR. **p <* 0.05; ***p* < 0.01; *****p <* 0.0001; ns: *p >* 0.05. 2-sided Mann-Whitney test.

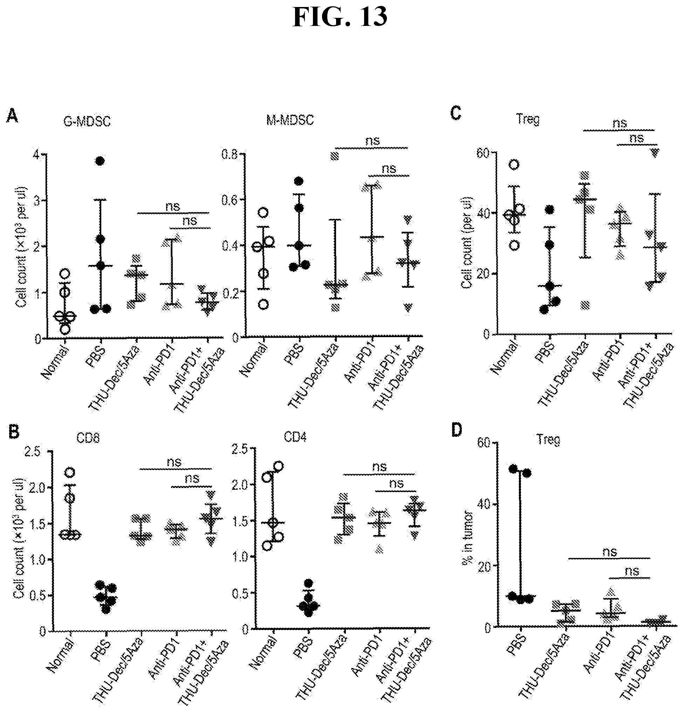

Figure 13. THU-Dec alternating with THU-5Aza further enhanced the immuno-therapeutic effects of anti-Pd1. C57BL/6 mice were inoculated via tail vein with LL3-luc lung cancer cells (0.35×10⁶cells/mouse). After documentation of lung invasion by live-imaging, mice (n = 5/group) were distributed to PBS, THU-Dec (10/0.1 mg/kg SC 2×/wk) followed by THU-5Aza SC 1×/wk, anti-Pd1 (5 mg/kg IP q5d) or anti-Pd1+THU-Dec/5Aza combination. A) Absolute numbers of peripheral blood G-MDSC and M-MDSC were significantly and similarly decreased by the different treatments vs vehicle. B) PB CD8⁺ and CD4⁺ T-cells were significantly and similarly increased by the different treatments vs vehicle. C) PB T_{reg} were non-significantly increased by the different treatments. D) T_{reg} infiltration of tumor was significantly and similarly decreased by all treatments vs vehicle. Bar and whiskers = median ± IQR. ns: *p* > 0.05. 2-sided Mann-Whitney test.

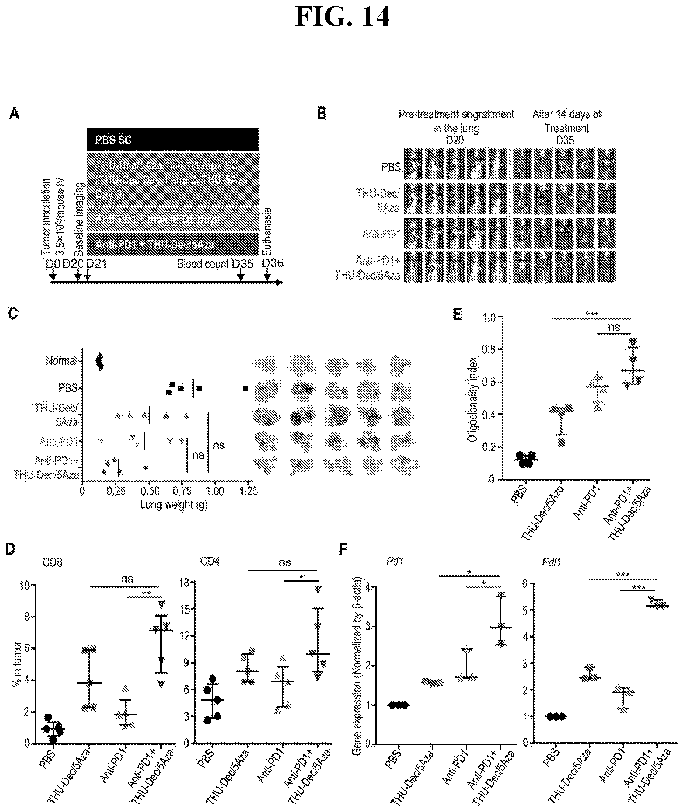

Figure 14: Alternating THU-Dec with THU-5Aza further enhanced the immuno-therapeutic effects of anti-Pd1. C57BL/6 mice were inoculated via tail vein with LL3-luc lung cancer cells (0.35×10⁶cells/mouse). After documentation of lung invasion by live-imaging, mice (n = 5/group) were distributed to PBS, THU-Dec/5Aza, anti-Pd1 or anti-Pd1+THU-Dec/5Aza combination. A) Experiment schema. B) Tumor burden before and after 14 days of treatment with the different regimens. Live-imaging. C) Tumor burden as measured by lung weights was lowest with the combination treatment, although not significantly so vs the other treatments. D) Effects of the different regimens on tumor infiltration by immune-effector cells. Measured by flow cytometry of homogenized tumor tissue. E) T-cell oligoclonality was increased the most by the combination treatment. Oligoclonality index = percentage of the overall T-cell population represented by the 1000 most abundant T-cell clones, measured by T-cell receptor (TCR) diversity analysis of TILs by RNA sequencing. F) Pd1/PD-L1 immune checkpoint gene expression in tumor tissue was increased the most by the combination treatment. Bar and whiskers = median ± IQR. **p < 0.05; **p <* 0.01; ****p <* 0.001; ns: *p >* 0.05. 2-sided Mann-Whitney test.

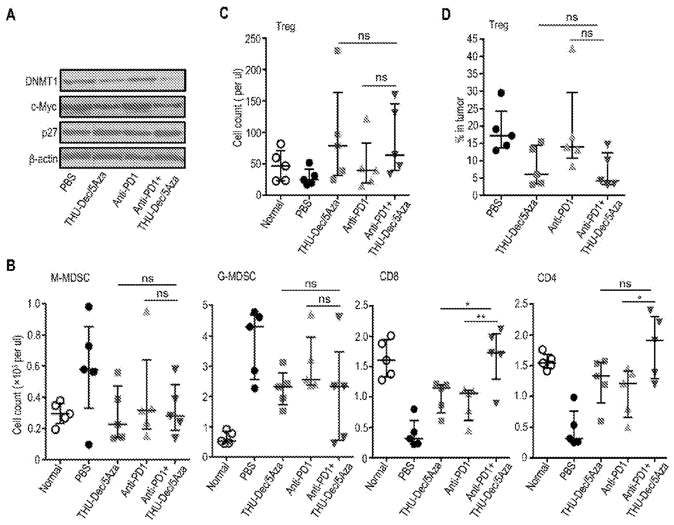

Figure 15. Alternating THU-Dec with THU-5Aza further enhanced the immuno-therapeutic effects of anti-Pd1. C57BL/6 mice were inoculated via tail vein with LL3-luc lung cancer cells (0.35×10⁶cells/mouse). After documentation of lung invasion by live-imaging, mice (n = 5/group) were distributed to PBS, THU-Dec/5Aza, anti-Pd1 or anti-Pd1+THU-Dec/5Aza combination. A) Combination epigenetic-immunotherapy produced more DNMT1-depletion, Myc reduction and p27 upregulation over THU-Dec/5Aza or ICB alone. Western blots of pooled tumor tissue harvested at euthanasia. B) Absolute numbers of peripheral blood G-MDSC and M-MDSC were significantly and similarly decreased by the different treatments vs vehicle, while PB immune-effector cells were most significantly increased by combination epigenetic-immunotherapy. C) PB T_{reg} numbers were not significantly altered by the treatments vs vehicle. D) T_{reg} infiltration of tumor was most decreased by combination treatment, but not to a significant extent vs the other treatments. Bar and whiskers = median ± IQR. **p <* 0.05; ***p <* 0.01; ns: *p >* 0.05. 2-sided Mann-Whitney test.

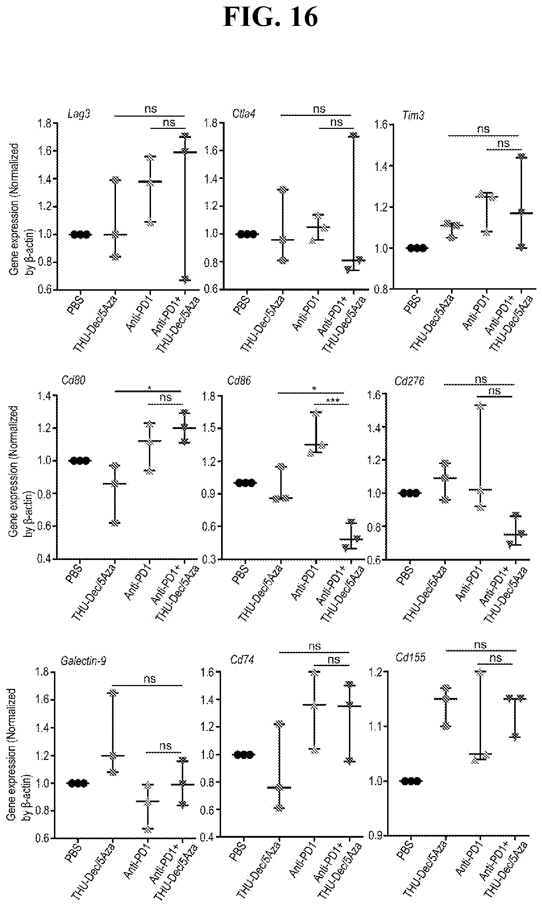

Figure 16. Effects of the epigenetic and anti-Pd1 treatment on immune checkpoint expression in tumor tissue and TILs. C57BL/6 mice were inoculated via tail vein with LL3-luc lung cancer cells (0.35×10⁶cells/mouse). After documentation of lung invasion by live-imaging, mice (n = 5/group) were distributed to PBS, THU-Dec/5Aza, anti-Pd1 or anti-Pd1+THU-Dec/5Aza combination. *Lag3, Ctla4,* and *Tim3* expression were measured in mRNA extracted from TILs (magnetically selected for CD8 expression), the other checkpoints were measured in mRNA isolated from whole tumor tissue. Bar and whiskers = median ± IQR. **p <* 0.05; ****p <* 0.001; ns: *p >* 0.05. 2-sided Mann-Whitney test.

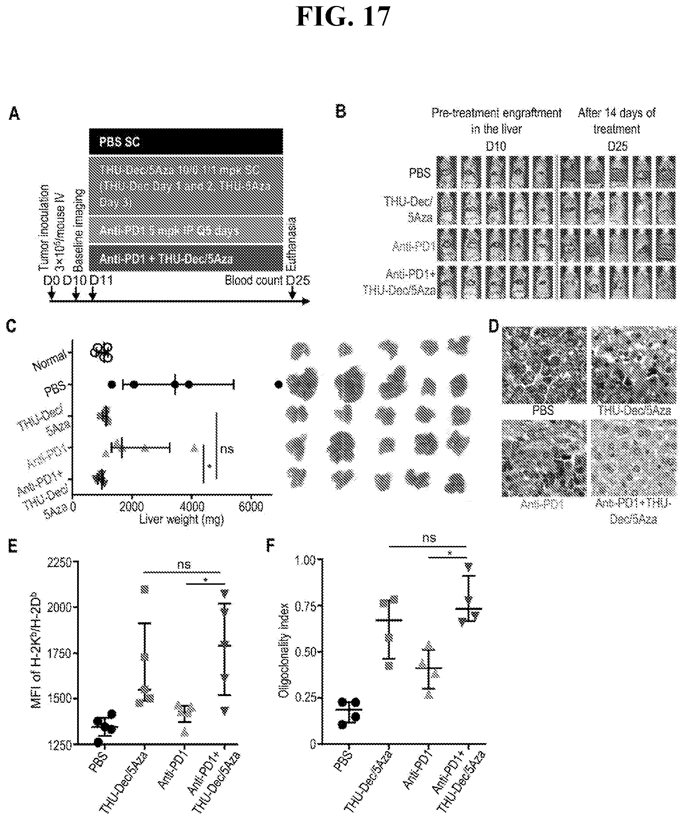

Figure 17: THU-Dec/5Aza epigenetic treatment was beneficial to the mice with SCLC, but ICB with anti-PD1 did not add benefit. B6/129 F1 mice were inoculated via tail vein with F1339-luc lung cancer cells (0.3×10⁶cells/mouse). After documentation of lung invasion by live-imaging, mice (n = 5/group) were distributed to PBS, THU-Dec/5Aza, anti-PD1 or combination anti-PD1+THU-Dec/5Aza. A) Experiment schema. B) Tumor burden before and after 14 days of treatment. Live-imaging. C) Tumor burden after 14 days of treatment measured by liver weights. 2-sided Mann-Whitney test. D) Tumor histological sections after 14 days of treatment. H&E staining, showing decreased nuclear-cytoplasmic ratio and increased necrosis with treatment. E) THU-Dec/5Aza or combination epigenetic-immunotherapy, but not anti-PD1 alone, increased H-2K^{b}/H-2D^{b} expression by tumor tissue. Measured by flow cytometry. F) The different treatments favored oligoclonality of TILs, with the greatest clonal restriction with combination epigenetic-immunotherapy. Oligoclonality index = percentage of the CD8+ T-cell population represented by the 1000 most abundant T-cell clones measured by TCR RNA-sequencing. Bar and whiskers = Median ± IQR. **p <* 0.05; ns: *p* > 0.05. 2-sided Mann-Whitney test.

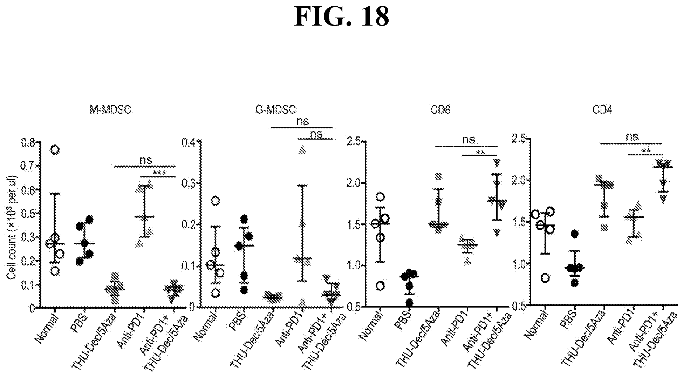

Figure 18. THU-Dec/5Aza alternating epigenetic treatment, ICB and combination epigenetic-ICB similarly decreased PB granulocytic and monocytic myeloid-derived suppressor cells (G-MDSC and M-MDSC), while peripheral blood CD8⁺ and CD4⁺ T-cells were significantly and similarly increased. B6/129 F1 mice were inoculated via tail vein with F1339-luc lung cancer cells (0.3×10⁶cells/mouse). After documentation of tumor invasion by live-imaging, mice (n = 5/group) were randomized to PBS, THU-Dec/5Aza, anti-Pd1 or combination anti-Pd1+THU-Dec/5Aza. Bar and whiskers = Median ± IQR. ***p <* 0.01; ****p* < 0.001; ns: *p >* 0.05. 2-sided Mann-Whitney test.

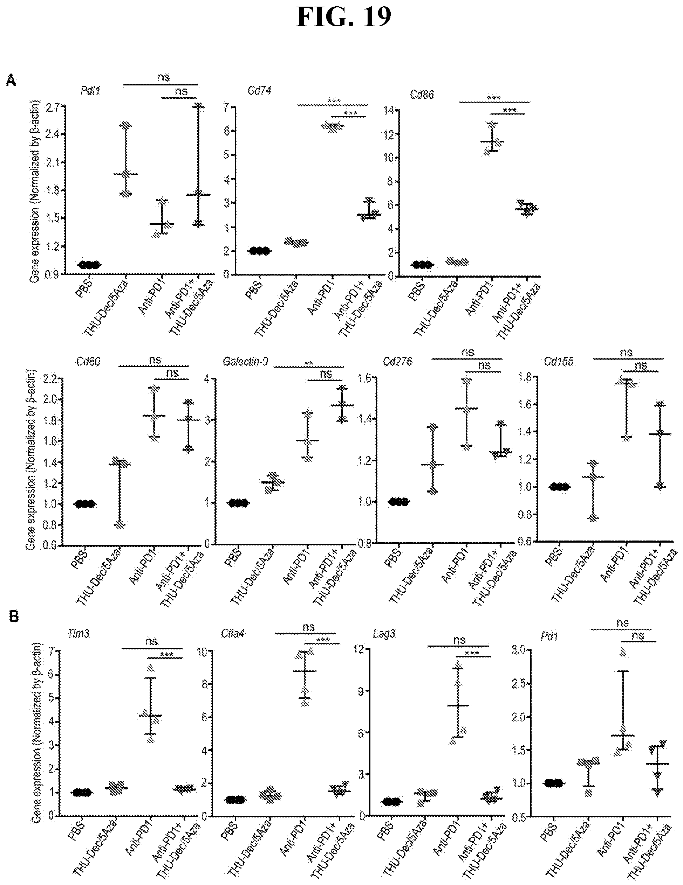

Figure 19. Several immune checkpoints other than Pd1/PD-L1 were upregulated in SCLC treated with anti-Pd1. B6/129 F1 mice were inoculated via tail vein with F1339-luc lung cancer cells (0.3×10⁶cells/mouse). After documentation of tumor invasion by live-imaging, mice (n = 5/group) were randomized to PBS, THU-Dec/5Aza, anti-Pd1 or combination anti-Pd1+THU-Dec/5Aza. A) Immune-checkpoint expression in SCLC tumor tissue after 14 days of treatment. QRT-PCR. Tumor tissue from 3 individual mice per treatment group. B) Immune-checkpoint expression in TILs. TILs from 4 individual mice per group (magnetically selected for CD8 expression). Gene expression by QRT-PCR. Bar and whiskers = median ± IQR. ***p <* 0.01; ****p <* 0.001; ns: *p >* 0.05. 2-sided Mann-Whitney test.

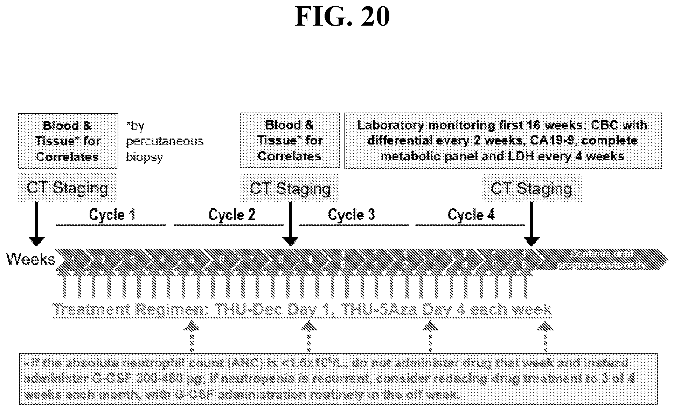

Figure 20 describes a first exemplary treatment of staggered decitabine and 5-azacytidine weekly treatment protocol for a human patient with cancer over a treatment period of multiple weeks. This protocol includes the possibility of skipping treatment a given week if absolute neutrophil count (ANC) is too low. In such instances, the patient can be administered G-CSF.
   The exemplary regimen includes the following. Oral THU at about 10 mg/kg administered 60-300 minutes before oral decitabine at about 0.16 mg/kg on day (e.g., Thu at breakfast, decitabine at lunch). Then, oral THU at about 10 mg/kg administered 60-300 minutes before oral 5-azazytidine at about 1.6 mg/kg on Day 4 (e.g., THU at breakfast, 5-azacytidine at lunch). If the AN is less than 0.5x10⁹/L, treatment is held for that week and G-CSF 200-480 ug is administered instead. If neutropenia is recurrent, consider routinely reducing drug treatment to 3 of 4 weeks each month, with G-CSF administration routinely in the week.

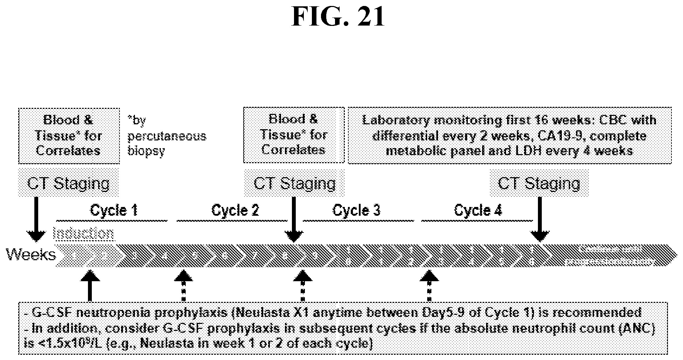

Figure 21 describes a second exemplary treatment staggered decitabine and 5-azacytidine weekly treatment protocol for a human patient with cancer over a treatment period of multiple weeks. In this protocol, there is an induction period where the patient is administered THU and then decitabine 60 minutes later for five consecutive days, and then G-CSF (NEULASTA neutropenia prophylaxis) anytime between days 5-9. The exemplary regimen includes the following. In week 1: i) oral Thu at about 10 mg/kg is administered 60 minutes before oral decitabine at about 0.2 mg/kg for 5 consecutive days, and ii) Neulasta neutropenia prophylaxis is recommended, to be administered anytime between Days 5-9 of the cycle. Week 2: if there are no greater than or equal to grade 3 treatment-related non-hematologic toxicities, oral Thu-decitabine is administered at the same dose in week 2 according to the following schema: i) baseline ANC in week 2 is 0.5-1.0x10⁹ /L, administer for 2 consecutive days; ii) baseline ANC in week 2 is greater than 1.0-1.5x10⁹ /L, administer for 3 consecutive days; iii) baseline ANC in week 2 is greater than 1.5-2.0x10⁹ /L, administer for 4 consecutive days; and iv) baseline ANC in week 2 is greater than 2.0x10⁹ /L, administer for 5 consecutive days. Weeks 3 and 4, if there are no greater than or equal to grade 3 treatment-related non-hematologic toxicities and the ANC is greater than 0.5x10⁹/L, administer oral Thu-decitabine at the same dose for 2 consecutive days. Induction cycles can be repeated if clinically indicated (e.g., in a patient with a partial response but who is still symptomatic and in whom the first induction cycle was well tolerated; or in a patient with disease previously controlled by induction then Maintenance but subsequently relapsing while on maintenance. Cycle 2 onward - maintenance cycles: Oral Thu about 10 mg/kg is administered 60 minutes before oral decitabine at about 0.2 mg/kg two times per week on consecutive days if there are no greater than or equal to grade 3 treatment-related non-hematologic toxicities and the ANC is greater than 0.5x10⁹/L (if the ANC is less than 0.5x10⁹/L, treatment is held until recovery above this threshold then resumed with a dose reduction.

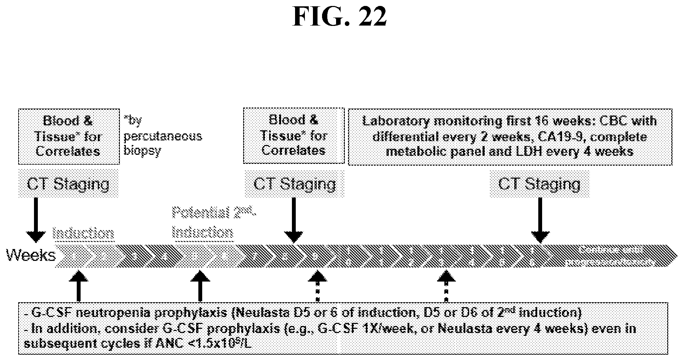

Figure 22 describes a third exemplary treatment staggered decitabine and 5-azacytidine weekly treatment protocol for a human patient with cancer over a treatment period of multiple weeks. This protocol has a first induction protocol and potential second induction protocol as described in Figure 22. The exemplary regimen includes the following. In week 1: i) oral Thu at about 10 mg/kg is administered 60 minutes before oral decitabine at about 0.2 mg/kg for 5 consecutive days, and ii) Neulasta neutropenia prophylaxis is recommended, to be administered anytime between Day 5 or 6 of the cycle. Week 2: if there are no greater than or equal to grade 3 treatment-related non-hematologic toxicities, oral Thu-decitabine is administered at the same dose in week 2 according to the following schema: i) baseline ANC in week 2 is 0.5-1.0x10⁹ /L, administer for 2 consecutive days; ii) baseline ANC in week 2 is greater than 1.0-1.5x10⁹ /L, administer for 3 consecutive days; iii) baseline ANC in week 2 is greater than 1.5-2.0x10⁹ /L, administer for 4 consecutive days; and iv) baseline ANC in week 2 is greater than 2.0x10⁹ /L, administer for 5 consecutive days. Weeks 3 and 4, if there are no greater than or equal to grade 3 treatment-related non-hematologic toxicities and the ANC is greater than 0.5x10⁹/L, administer oral Thu-decitabine at the same dose for 2 consecutive days. Cycle 2 - potentially a second induction cycle versus a maintenance cycle. Repeat induction as per the schema above if CA19-9 is not decreased by at least 10% from baseline or if clinically indicated, otherwise commence maintenance. Induction cycles can be repeated even after cycle 2 if the treating clinical team judges such a repeat to be in the best interest of the patient (e.g., for previously controlled disease relapsing during maintenance). Cycles 2 and 3 onward - maintenance cycles: Oral Thu about 10 mg/kg is administered 60 minutes before oral decitabine at about 0.2 mg/kg two times per week on consecutive days if there are no greater than or equal to grade 3 treatment-related non-hematologic toxicities and the ANC is greater than 0.5x10⁹/L (treatment is held until recovery of ANC to greater than 0.5x10⁹/L).

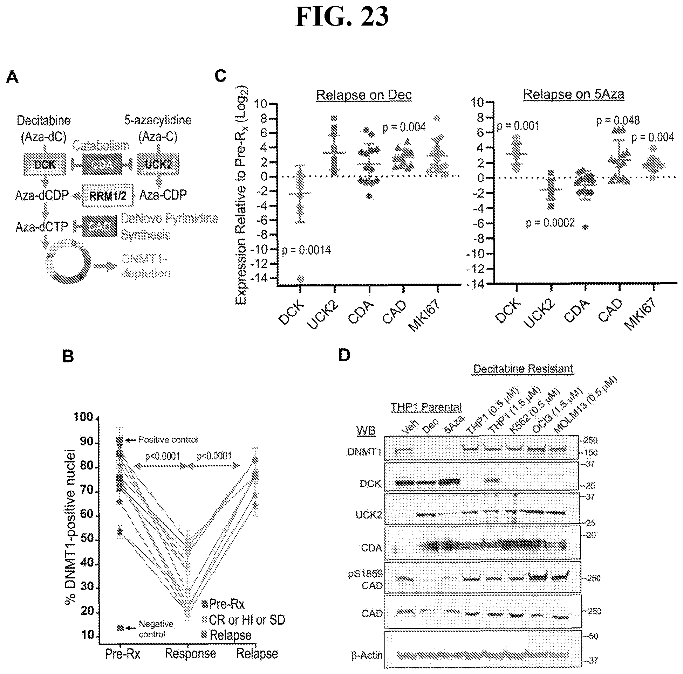

Figure 23 shows DNMT1 is not depleted at clinical relapse or with in vitro resistance. A) Key pyrimidine metabolism enzymes shown to be critical to DNMT1-depleting activity of decitabine (Dec) or 5-azacytidine (5Aza) in vitro. dCDP = deoxycytidine diphosphate; CDP = cytidine diphosphate; dCTP - deoxycytidine triphosphate. B) Decitabine (Dec) or 5-azacytidine (5Aza) decreased bone marrow DNMT1 at response (green) vs pre-treatment (dark blue) but not at relapse (red). Serial bone marrow biopsies from the same patient were cut onto the same slide, stained for DNMT1, and the number of DNMT1-positive nuclei was quantified objectively using ImageIQ software in 13 patients and positive/negative controls (tissue blocks of HCT116 wild-type and DNMT1-knockout cells respectively). D = days of therapy. Pre-Rx = pre-treatment; HI = hematologic improvement; CR = complete remission; SD = stable disease; Rel. = relapse. Mean±SD of x3 image segments (cellular regions) per sample; p-value paired t-test, 2-sided. C) Expression of key pyrimidine metabolism enzymes at relapse/progression on Dec. Schema shows key enzymes that favor (green) or impede (red) Dec or 5Aza conversion into the DNMT1-depleting Aza-dCTP. Bone marrow cells aspirated pre-treatment and at relapse/progression on Dec (13 patients, median duration of therapy 175 days, range 97-922) or 5Aza (14 patients, median duration of therapy 433 days, range 61-1155) were analyzed by QRT-PCR. Mean±SD technical replicatesx3. Paired t-test, 2-sided. D) Expression by Western blot of DNMT1 and key pyrimidine metabolism enzymes in Dec-resistant AML cells (THP1, K562, OCI-AML3 and MOLM13), and in parental THP1 AML cells treated with vehicle, Dec or 5-azacytidine (5Aza). Western blots.

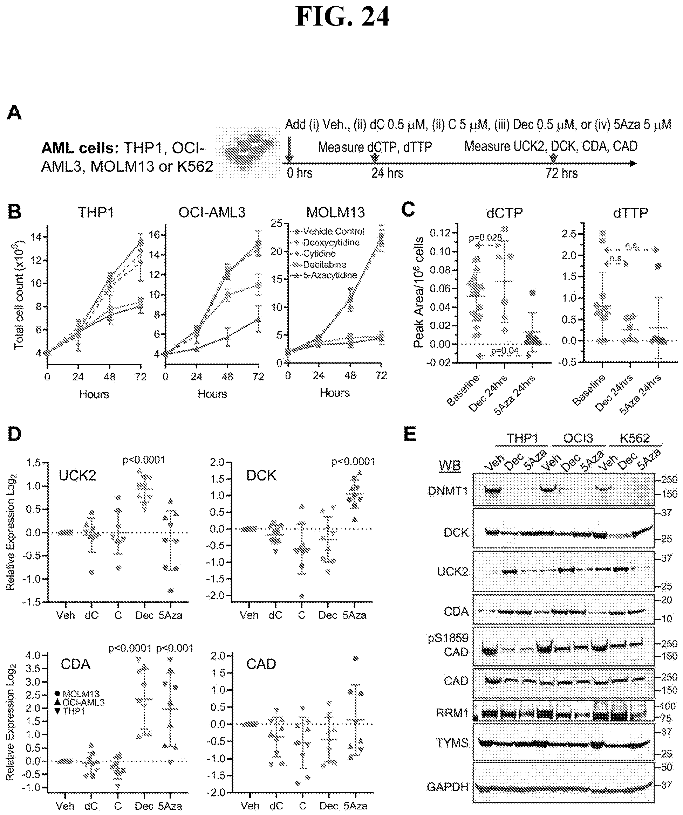

Figure 24 shows Dec and 5Aza cause nucleotide imbalances and metabolic compensations. A) Experiment schema. Vehicle, natural deoxycytidine (dC) 0.5 uM, natural cytidine (C) 5 uM, Dec 0.5 uM, or 5Aza 5 uM were added once to AML cells at 0 hours. B) Cell counts. By automated counter. Means±SD for 3 independent biological replicates for each cell line. C) Dec and 5Aza have opposite effects on dCTP levels. Measured by LCMS/MS 24 hrs after addition of Dec or 5Aza. Analyses of 2 or more independent nucleotide extractions from 3 different AML cells lines. Means±SD; p-values paired t-test, 1-sided. D) Gene expression 72 hours after Dec or 5Aza. Gene expression by QRT-PCR, relative to average expression in vehicle-treated controls. Means±SD for 3 independent biological replicates in each of 3 AML cell lines; p-values unpaired t-test vs vehicle, 2-sided. E) Western blot 72 hours after Dec or 5Aza. AML cells THP1, OCI-AML3 and K562. Western blots were reproduced in three independent biological replicates.

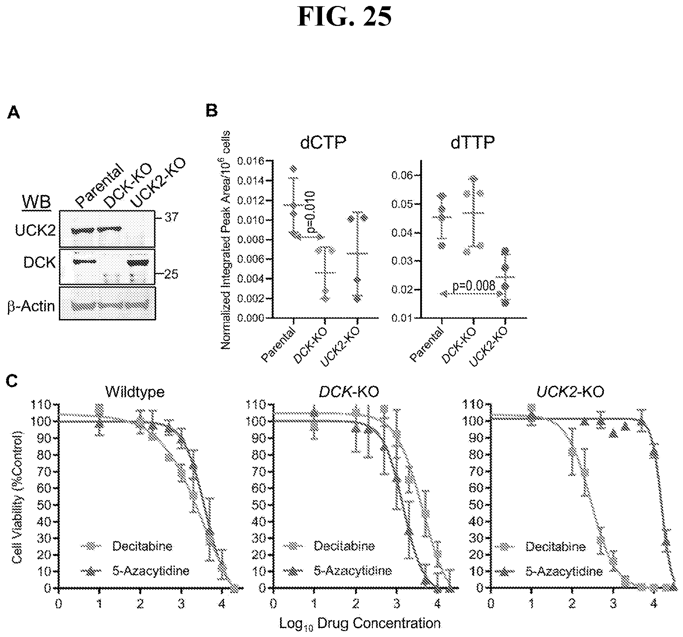

Figure 25 shows DCK is important for maintaining dCTP and UCK2 for maintaining dTTP levels. A) DCK and UCK2 knockout (KO) were confirmed by Western blot. HAP1 leukemia cells, KO by CRISPR-Cas9. B) DCK-KO lowers dCTP and UCK2-KO lowers dTTP. Analysis of independent nucleotide extractions. Means±SD; p-values unpaired t-test, 2-sided. C) Sensitivity of Wildtype, DCK-KO and UCK2-KO HAP1 leukemia cells to Dec vs 5Aza. Means±SD of 3 independent biological replicates.

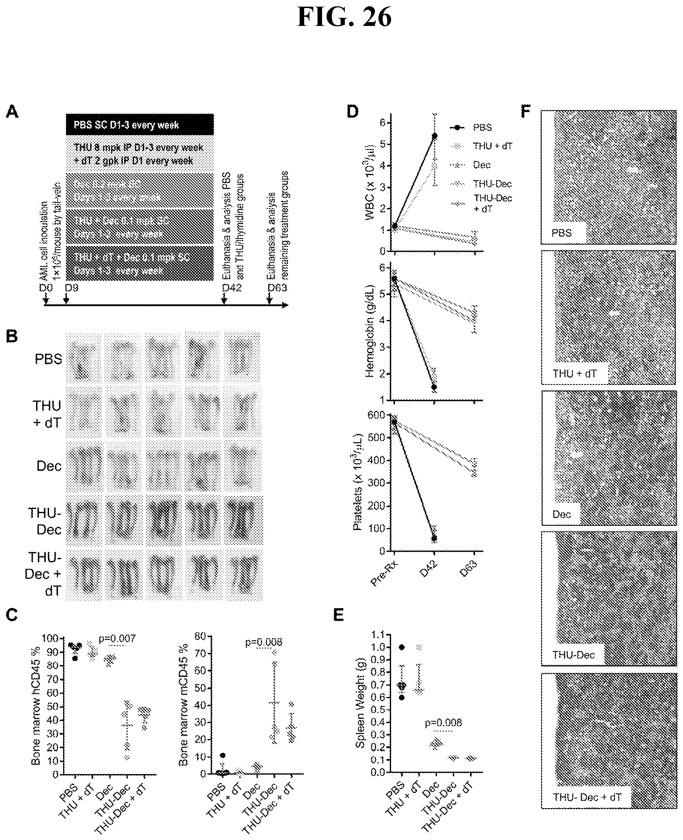

Figure 26 shows Impact on efficacy of adding CDA and/or de novo pyrimidine synthesis inhibitors. NSG mice were tail-vein inoculated with patient-derived AML cells (1×106cells/mouse) and randomized to (i) PBS vehicle control; (ii) CDA-inhibition by intra-peritoneal (IP) tetrahydrouridine (THU) and de novo pyrimidine synthesis inhibition by IP thymidine (dT); (iii) Dec; (iv) THU and Dec; (v) THU, Dec and dT (n=5/group). PBS, THU+dT mice were euthanized for distress on D42, and other mice were sacrificed for analysis on D63. A) Experiment schema. B) Femoral bones (from 2 of 5 mice/group). White = leukemia replacement, reddish = functional hematopoiesis. C) Bone marrow human (hCD45) and murine (mCd45) myelopoiesis content. Flow-cytometry. Median±IQR. p-value Mann-Whitney test 2-sided. D) Blood counts before treatment and at euthanasia/sacrifice. Measured by Hemavet. Median±IQR. E) Spleen AML burden as measured by spleen weights at euthanasia/sacrifice. Median±IQR. p-value Mann-Whitney test 2-sided. F) Spleen histology. Hematoxylin-Eosin stain of paraffin-embedded sections. Magnification 400X. Leica DMR microscope.

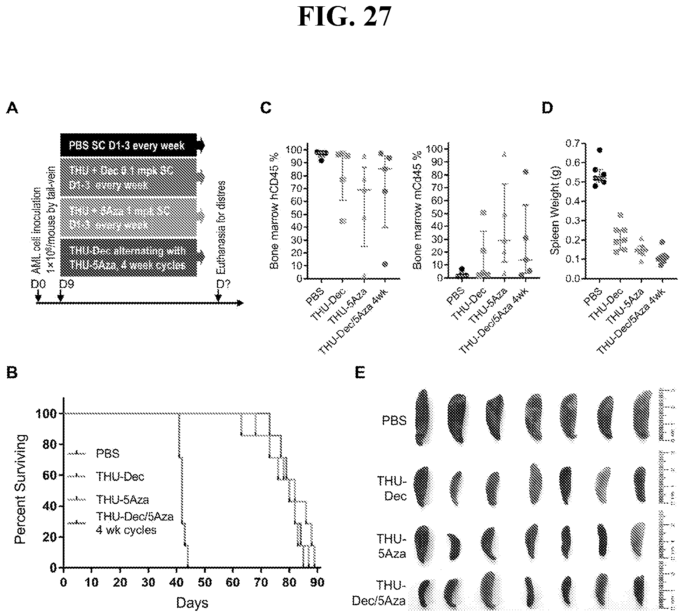

Figure 27 shows Comparison of THU/Dec alone vs THU/5Aza alone vs THU/Dec alternating with THU/5Aza in 4 week cycles. NSG mice were tail-vein inoculated with patient-derived AML cells (1×106cells/mouse) and on Day 9 after inoculation randomized to the treatments as shown (n=7/group). Mice were euthanized if there were signs of distress. A) Experiment schema; B) Time-to-distress and euthanasia. C) Bone marrow human (hCD45) and murine (mCd45) myelopoiesis content. Femoral bones flushed after euthanasia. Measured by flow-cytometry. Median±IQR. D) Spleen weights at time-of-distress/euthanasia. Median±IQR. E) Spleens at the time-of-distress/euthanasia.

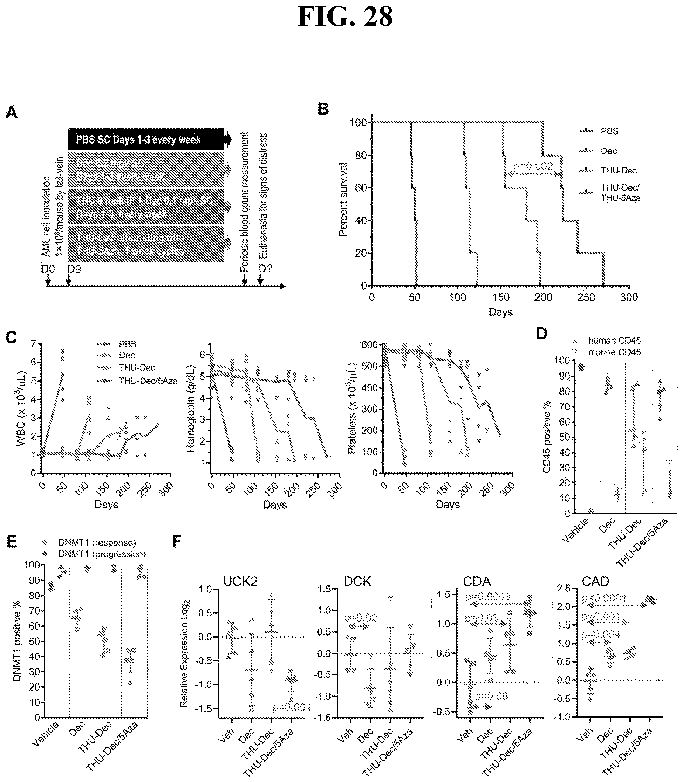

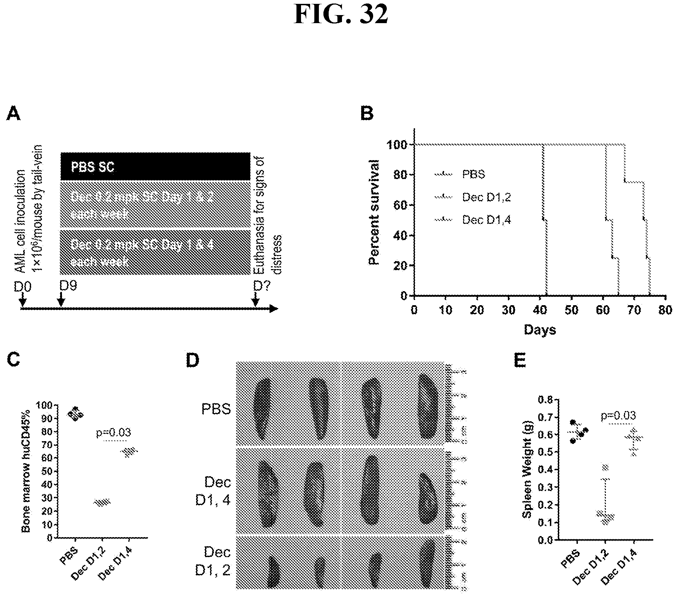

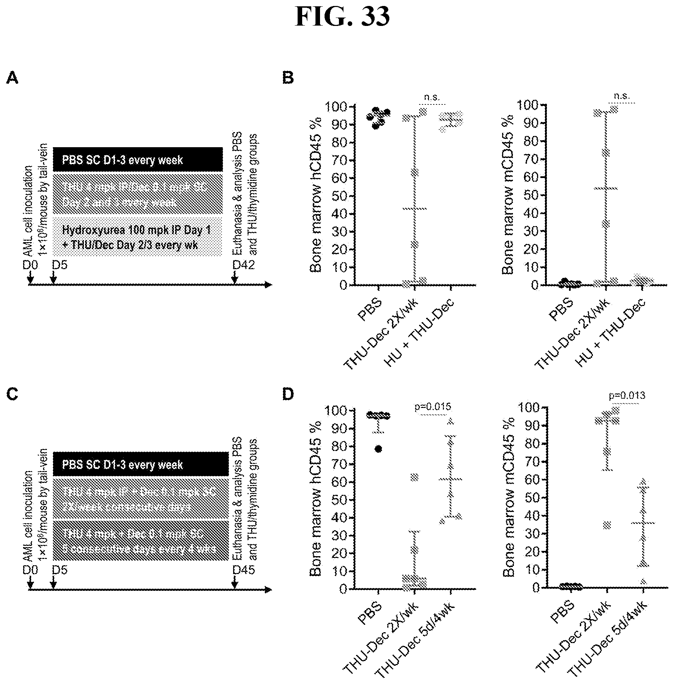

Figure 28 shows Alternating THU/Dec with THU/5Aza week to week. NSG mice were tail-vein inoculated with patient-derived AML cells (1×106cells/mouse) and randomized to the treatments shown (n=5/group). Blood counts were obtained periodically by tail-vein phlebotomy. Mice were euthanized for signs of distress. A) Experiment schema; B) Time-to-distress. Log-rank test. C) Serial blood counts. Measured by Hemavet. Median±IQR. D) Bone marrow replacement by AML. Bone marrow human and murine CD45+ cells measured by flow-cytometry (Figure 32) after euthanasia (time-points panel B). Median±IQR. p-value Mann-Whitney test 2-sided. E) DNMT1 was not depleted from AML cells at progression (time-points panel B) but was depleted at time-of-response (bone marrow harvested at Day 63 in a separate experiment). Flow cytometry (Figure 33). F) Pyrimidine metabolism gene expression in bone marrow AML cells. QRT-PCR using human gene specific primers, bone marrow harvested after euthanasia. p-values vs vehicle, unpaired t-test, 2-sided.

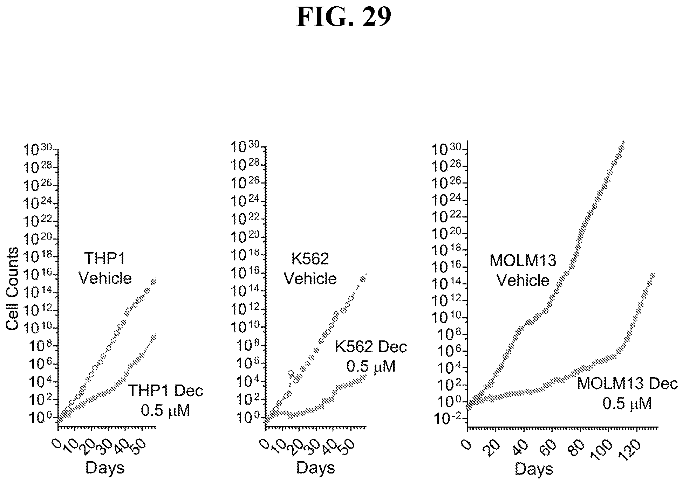

Figure 29 shows time to emergence of AML cells exponentially proliferating in presence of clinically relevant concentrations of decitabine (Dec). Cell counts by automated counter.

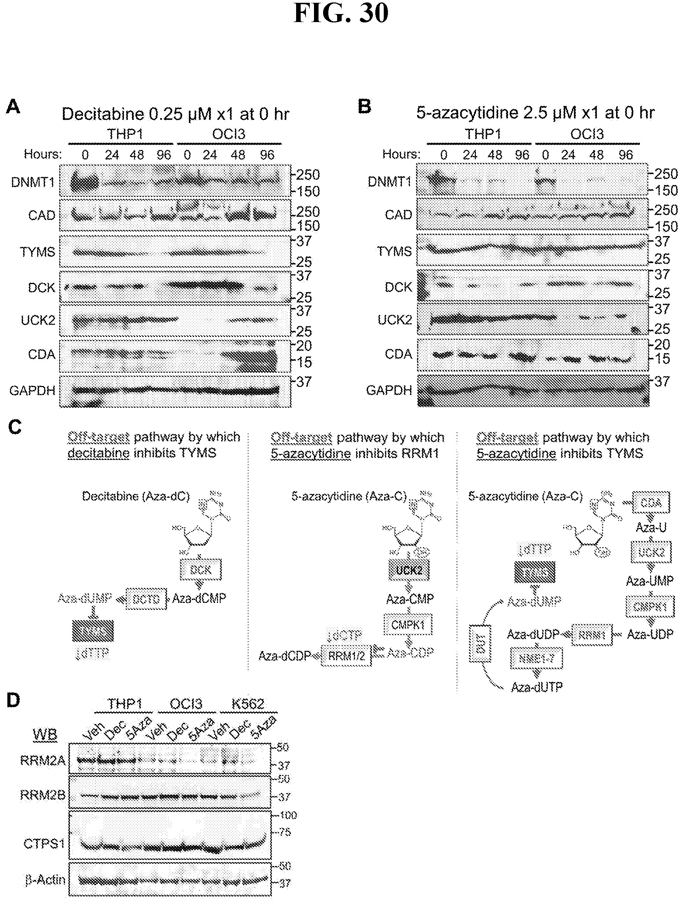

Figure 30 shows a time-course analysis suggested that peak changes in pyrimidine metabolism enzyme protein levels occurred between 48-96 hours after a single exposure to decitabine (Dec) 0.25 µM or 5-azacytidine (5Aza) 2.5 uM. A) Western blots for DNMT1, UCK2, DCK, CDA, CAD, TYMS and GAPDH before and up to 96 hours after addition of decitabine (Dec) 0.25 µM or B) 5-azacytidine 2.5 uM to THP1 and OCI-AML3 cells (added once at 0 hours). C) Off-target pathways of Dec and 5Aza. D) Effects of Dec and 5Aza on RRM2A, RRM2B and CTPS1 protein levels. Western blots 72 hours after addition of a single dose of Dec 0.25 µM or 5Aza 2.5 uM. Western blots were reproduced in biological replicates.

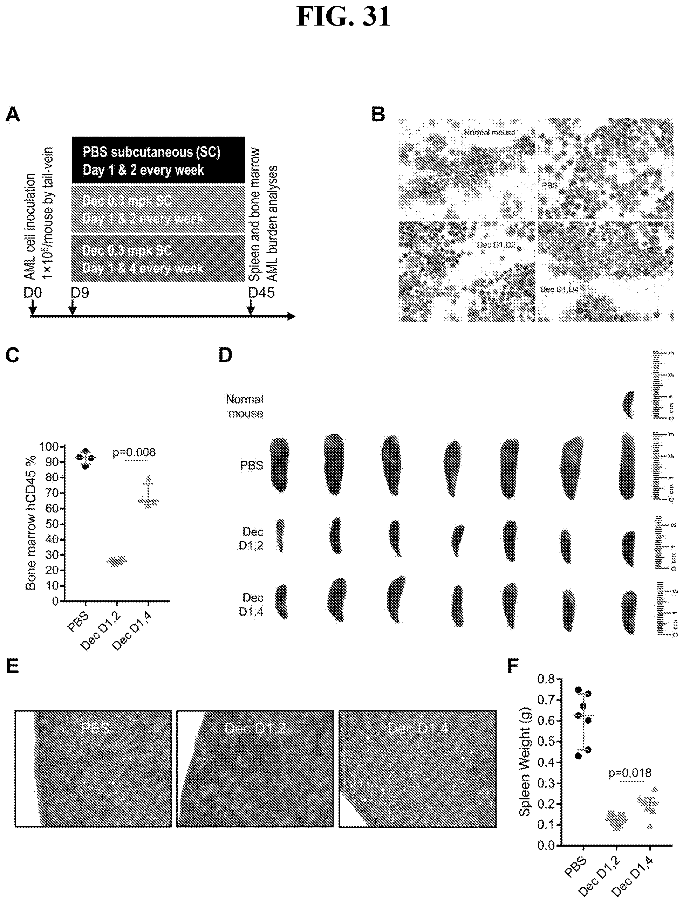

Figure 31 shows Impact of decitabine scheduling to avoid vs coincide with DCK troughs. NSG mice were tail-vein inoculated with patient-derived AML cells (1×10⁶cells/mouse) and randomized on day 9 after inoculation to (i) PBS vehicle control; (ii) subcutaneous (SC) decitabine (Dec) 0.3 mg/kg (mpk) on Day 1 and 2 of each week (D1,2); (iii) Dec 0.3 mpk on Day 1 and 4 of each week (D1,4)(n=7/group). Mice were euthanized/sacrificed on day 45 when PBS treated mice showed signs of distress. A) Experiment schema; B) Bone marrow cell cytospin and Giemsa-stain at Day 45. Normal = normal NSG mouse bone marrow; Leica DMR microscope, 630X. C) Percentage of human CD45+ (huCD45) positive cells in bone marrow. Flow cytometry. Median ± IQR. p-value Mann-Whitney test 2-sided. 5 mice in each treatment group analyzed. D) Spleens at Day 45. Normal = spleen from normal NSG mouse. E) Spleen histology. Hematoxylin-Eosin stain of paraffin-embedded sections. Leica DMR microscope, 400X.F) Spleen weights. Median±IQR. p-value Mann-Whitney test 2-sided.
Figure 32 shows impact of decitabine scheduling to avoid or coincide with DCK troughs. NSG mice were tail-vein inoculated with patient-derived AML cells (1×10⁶cells/mouse) and on day 9 after inoculation randomized to (i) PBS vehicle control; (ii) subcutaneous (SC) decitabine (Dec) 0.2 mg/kg (mpk) on Day 1 and 2 of each week (D1,2); (iii) Dec 0.2 mpk on Day 1 and 4 of each week (D1,4)(n=4/group). Mice were euthanized for signs of distress. A) Experiment schema; B) Time-to-distress. p-value Log-rank test. C) Bone marrow human leukemia cell burden at time-of-distress. Flow cytometry for human CD45+ cells. Median ± IQR. p-value Mann-Whitney test 2-sided. D) Spleens at time-of-distress. E) Spleen weights at time-of-distress. Median ± IQR. p-value Mann-Whitney test 2-sided.
Figure 33 shows The addition of hydroxyurea to inhibit ribonucleotide reductase did not augment THU-decitabine activity; Distributed administration of THU-decitabine 2X/week was superior to pulse-cycled administration for 5 consecutive days every 4 weeks. NSG mice were tail-vein inoculated with patient-derived AML cells (1×10⁶ cells/mouse) and on Day 5 after inoculation randomized to the treatments as shown (n=7/group). Mice were euthanized if there were signs of distress. A) Experiment schema to evaluate potential benefit of adding hydroxyurea to inhibit ribonucleotide reductase; B) Bone marrow human (hCD45) and murine (mCd45) myelopoiesis content. Femoral bones flushed after termination of the experiment at the time PBS-treated mice developed signs of distress. Measured by flow-cytometry. Median±IQR. P-value 2-sided Mann-Whitney test. n.s. = not significant. C) Experiment schema to compare metronomic administration 2X/week versus pulse-cycled administration for 5 consecutive days every 4 weeks; D) Bone marrow human (hCD45) and murine (mCd45) myelopoiesis content. Femoral bones flushed after termination of the experiment at the time PBS-treated mice developed signs of distress. Measured by flow-cytometry. Median±IQR. P-value 2-sided Mann-Whitney test. n.s. = not significant.

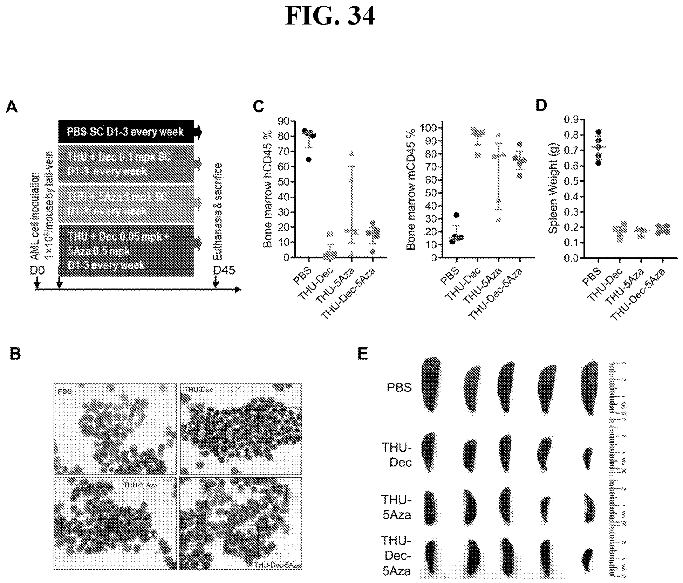

Figure 34 shows THU/decitabine vs THU/5-azacytidine vs THU/decitabine/5-azacytidine. NSG mice were tail-vein inoculated with patient-derived AML cells (1×10⁶cells/mouse) and on Day 9 after inoculation randomized to the treatments as shown (n=5/group). All mice were euthanized or sacrificed when the vehicle-treated group became distressed at Day 45. A) Experiment schema; B) Giemsa stained cytospins of bone marrow cells. Flushed from femoral bones after euthanasia. Magnification 630X. Leica DMR microscope. C) Bone marrow human (hCD45) and murine (mCd45) myelopoiesis content. Measured by flow-cytometry. Median±IQR. D) Spleen weights at time-of-distress/euthanasia. Median±IQR. E) Spleens.

### DETAILED DESCRIPTION

The invention is set out in the appended claims. The embodiments of the description which do not fall within the scope of said claims are provided for illustrative purposes only and do not form part of the present invention. Provided herein are compositions, systems, kits, and methods for treating a patient with cancer by alternate administration of decitabine and 5-azacytidine, in particular as per the treatment regime as defined in claim 1. Such administration is combined with an inhibitor of the enzyme cytidine deaminase (e.g., tetrahydrouridine) that otherwise rapidly catabolizes decitabine and 5-azacytidine. The time between cycles of decitabine and 5-azacytidine administration is about three to four days as defined in claim 1. The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

The benefits of immune checkpoint blockade (ICB) to treat lung cancer are limited to a minority of patients, with some cancers demonstrating paradoxical hyper-growth. There is thus a need for complementary treatments that cytoreduce cancers but spare immune-effectors. Inhibition of the epigenetic regulator DNA methyltransferase 1 (DNMT1) has been validated to do this while also increasing cancer immune-visibility. Moreover, the clinical pro-drugs decitabine and 5-azacytidine, after processing by pyrimidine metabolism into a nucleotide analog, can deplete DNMT1. Unfortunately, clinical trials combining these pro-drugs with ICB have produced disappointing results. Metabolic control mechanisms sense and preserve nucleotide balances. Therefore, work conducted during development of embodiments herein examined reactions of the pyrimidine metabolism network in lung cancer cells to decitabine and 5-azacytidine inputs and found that expression of enzymes essential to nucleotide conversion shifted adaptively, automatically dampening DNMT1-depletion. However, responses to the deoxyribonucleoside decitabine primed for activity of the ribonucleoside 5-azacytidine and vice versa. Thus, in murine models of disseminated non-small cell and small cell lung cancer, alternating decitabine with 5-azacytidine, timed to bypass auto-dampening and exploit cross-priming instead, combined with an inhibitor of the enzyme cytidine deaminase that rapidly catabolizes decitabine/5-azacytidine in vivo, significantly increased tumor DNMT 1-depletion, cytoreduction, antigen presentation, T-cell infiltration/oligoclonality, and time-to-distress, without off-target cytotoxic effects. Then, combination with ICB produced complete tumor regressions that resisted tumor re-challenge ('immune-memory').

There numerous advantages to the systems and methods described herein. Metabolic control mechanisms sense and preserve nucleotide balances. Work conducted during development of embodiments herein examined pyrimidine metabolism reactions to nucleoside inputs and discovered that expression of enzymes essential to nucleoside uptake shifted adaptively to dampen drug uptake. Fortuitously, however, responses to the deoxyribonucleoside decitabine primed for uptake of the ribonucleoside 5-azacytidine and vice versa. Thus, in murine models of disseminated non-small cell and small cell lung cancer, alternating decitabine with 5-azacytidine, timed to bypass auto-dampening and exploit cross-priming, combined with an inhibitor of the enzyme cytidine deaminase that otherwise rapidly catabolizes decitabine/5-azacytidine, significantly increased tumor DNMT1-depletion, antigen presentation, T-cell infiltration/oligoclonality, cytoreduction and time-to-distress. Specifically, decitabine alone was ineffective in a pre-clinical in vivo model of cancer localized to the liver, but co-administration of lower doses of decitabine with an inhibitor of CDA, tetrahydrouridine (THU), eliminated cancer cell sanctuary in CDA-rich liver, importantly, without bone marrow cytotoxicity. Alternating decitabine with 5-azacytidine and combination of both with an inhibitor of CDA, significantly enhanced DNMT1-depletion from NSCLC and SCLC cells in vivo. Regimens optimized in this way preserved a non-cytotoxic mechanism-of-action that spared immune-effectors and synergized with ICB, to produce complete tumor regressions that resisted tumor re-challenge. The optimized DNMT1-depleting regimens produced greater upregulation of antigen presenting MHC class I molecules (MHC I H-2Kb/H-2Db) and also cancer-testis antigens (Magea1-a4) (that are displayed in MHC class I), and infiltration of tumor by cytotoxic T-cells.

### EXAMPLES

### EXAMPLE 1

### Combination epigenetic-immunotherapy generates anti-cancer immune memory in murine models of lung cancer

This Example describes time delayed dosing of decitabine and 5-azacytidine for the treatment of lung cancer in a mouse model.

### RESULTS

### Pyrimidine metabolism enzymes that determine decitabine and 5-azacytidine sensitivity

To identify pyrimidine metabolism enzymes most relevant to the activity of decitabine and 5-azacytidine in cells, the expression of 45 known pyrimidine metabolism enzymes and transporters were examined for correlations with decitabine or 5-azacytidine sensitivity (concentrations of decitabine or 5-azacytidine needed to produce 50% growth inhibition [GI50] of cancer cell lines in the NCI60 panel). Growth inhibition by decitabine (lower GI50) correlated most strongly with expression of thymidylate synthase (TYMS) (Spearman correlation coefficient [r] = -0.42, p = 0.0008), deoxycytidine deaminase (DCTD) (r = -0.42, p = 0.0009) and DCK (r = -0.34, p = 0.009) (Figure 1A). DCK phosphorylates decitabine into a deoxycytidine monophosphate (dCMP) analog, Aza-dCMP - in the 'on-target' DNMT1-depleting pathway, Aza-dCMP is phosphorylated twice more into Aza-dCTP that depletes DNMT1 (Figure 1C) (14). In an 'off-target' pathway, DCTD deaminates some of this Aza-dCMP into a deoxyuridine monophosphate (dUMP) analog, Aza-dUMP, that potently inhibits TYMS (17-19), an action that decreases deoxycytidine triphosphate (dTTP) (TYMS supplies cells with dTTP). The decrease in dTTP in turn has been shown to increase deoxycytidine triphosphate (dCTP) by dis-inhibiting ribonucleotide reductase (17-19) (Figure 1C).

Growth inhibition by 5-azacytidine most strongly correlated with expression of TYMP (r=-0.34, p = 0.009), NT5C (r = -0.3, p = 0.022) and UCK2 (r = -0.28, p = 0.029) (Figure 1D, E). (14). UCK2 phosphorylates 5-azacytidine into a cytidine monophosphate (CMP) analog, Aza-CMP; NT5C and TYMP in tandem can convert some of this Aza-CMP into a pyrimidine analog that has been reported to inhibit de novo pyrimidine synthesis, an 'off-target' effect that decreases dCTP (20). In the on-target pathway, Aza-CMP is phosphorylated to a cytidine diphosphate (CDP) analog, Aza-CDP, that is reduced by ribonucleotide reductase to Aza-dCDP, that is phosphorylated into the Aza-dCTP that depletes DNMT1 (Figure 1C). The on-target pathway incorporates an off-target effect of partial inhibition of ribonucleotide reductase (21) (Figure 1C), which also decreases dCTP (21).

In keeping with a drug efficacy requirement of incorporating into DNA, growth inhibition by both decitabine and 5-azacytidine significantly inversely correlated with cell doubling-time (r = 0.30, p = 0.021 and r = 0.29, p = 0.024 respectively) (Figure 1B, E). Linking these observations to clinical practice, myeloid malignancies that are currently the only approved indication for decitabine or 5-azacytidine, express several-fold higher levels of DCK and UCK2 at baseline, and have a higher growth fraction, than lung cancers (Figure 9).

### Adaptive responses of the pyrimidine metabolism network to decitabine or 5-azacytidine inputs

Thus, decitabine and 5-azacytidine, despite a common on-target action of DNMT1-depletion, have separate off-target actions that drive cellular dCTP levels in opposite directions (Figure 1C, 2A) (16-19, 21). To evaluate if these distinct actions induce specific adaptive responses by the pyrimidine metabolism network that might impact drug metabolism, expression of Dck, Uck2, Cda and Cad were measured using serial QRT-PCR and Western blots in a murine p53-null SCLC cell line (F1339) treated in vitro with DMSO (vehicle), decitabine 0.5 µM, 5-azacytidine 5 µM, natural deoxycytidine 0.5 µM, or natural cytidine 5 µM (F1339 cells were derived from a tumor established by intra-tracheal instillation of Adeno-CMV-Cre in Rb^{lox}/p53^{lox} mice (22)). Decitabine and 5-azacytidine, but not vehicle, natural deoxycytidine or natural cytidine, significantly decreased F1339 proliferation (Figure 2B). Decitabine and 5-azacytidine, but not vehicle, natural deoxycytidine or cytidine, induced rapid, consistent and distinct changes in expression of these enzymes: decitabine significantly increased Uck2, Cda and Cad expression several-fold, while 5-azacytidine increased Dck, Cda and Cad expression several-fold, with mRNA expression changes peaking at ~72 hours after drug exposure (Figure 2C). Protein levels tracked the mRNA changes and peaked at ~96 hours after drug exposure (Figure 2D).

### p53-null F1339 cells are chemorefractory but sensitive to decitabine and 5-azacytidine

F1339 cells expressing luciferase (F1339-luc) were inoculated via tail vein into B6/129 F1 mice, and after confirmation of tumor engraftment by bioluminescent imaging, the mice commenced treatment with high doses of etoposide and cisplatin, which are used to treat SCLC clinically (Figure 3A). This aggressive DNA-damaging/apoptosis-based treatment did not benefit the mice - there was no tumor cytoreduction (Figure 3A) or survival gain to the mice receiving this treatment vs vehicle-treated mice (Figure 3B). Similarly, in vitro, a clinically relevant concentration of the DNA damaging/apoptosis-inducing deoxycytidine analog cytarabine (Ara-C, 0.5 µM) did not reduce proliferation of the p53-null SCLC cells (Figure 3C). In contrast, concentrations of the deoxycytidine analog decitabine equimolar to that of cytarabine, and 5-azacytidine at 5 µM (a clinically relevant and safe concentration of this ribonucleotide analog), did significantly impede the SCLC cell proliferation (Figure 3C). These anti-proliferative actions of decitabine and 5-azacytidine were not by apoptosis-induction, evidenced by unaltered cell membrane Annexin-V staining 24 hours after initiation of drug exposure (Figure 3D). Instead, decitabine and 5-azacytidine treatment, but not cytarabine, induced morphologic changes in the cells consistent with differentiation induction (decreased nuclear cytoplasmic ratio, increased cytoplasmic complexity) (Figure 3E). Also consistent with a differentiation-based mechanism-of-action, decitabine and 5-azacytidine, but not cytarabine, decreased Myc protein, the master transcription factor regulator of cell growth and division, and simultaneously increased p27/CDKN1B protein, the canonical cyclin-dependent kinase inhibitor that mediates cell cycle exits by differentiation (Figure 3F).

### Addition of the CDA-inhibitor THU to decitabine or 5-azacytidine enhanced lung cancer Dnmt1-depletion, cytoreduction and antigen presentation in vivo

The catabolic enzyme CDA deaminates decitabine and 5-azacytidine into uridine nucleoside analogs that do not deplete DNMT1 but instead cause off-target anti-metabolite effects (Figure 1C). CDA is highly expressed at baseline in many normal solid organs (e.g., lungs), forming an enzyme barrier impeding activity of decitabine and 5-azacytidine in these tissues in vivo (14). CDA is also rapidly induced by exposure of cancer cells to decitabine (Figure 2). We therefore evaluated the utility of adding the CDA-inhibitor tetrahydrouridine (THU) to decitabine or 5-azacytidine for in vivo therapy of lung cancer. A murine syngeneic model of disseminated lung cancer was established by tail-vein inoculation of C57/BL6 mice with Lewis lung tumor cells (LL3). At day 21 after tumor inoculation, mice were randomized to treatment with: (i) vehicle; (ii) decitabine; (iii) 5-azacytidine; (iv) THU and decitabine; or (v) THU and 5-azacytidine (Figure 4A). The drugs were administered by regimens that are not cytotoxic to bone marrow as we have previously described (9, 26, 29). Decitabine or 5-azacytidine increased time-to-distress of the mice by about 20 days over vehicle, and the addition of THU significantly increased this to 30 days for both decitabine and 5-azacytidine (Figure 4B). Tumor tissues harvested at the time-of-euthanasia were analyzed for achievement of the intended molecular pharmacodynamic effect of Dnmt1-depletion. Dnmt1 protein levels were lowest in the tumor tissues from mice treated with THU-decitabine or THU-5-azacytidine (Figure 4C). Similarly, the greatest decreases in Myc protein (Figure 4C), and greatest increases in p27/Cdkn1b (Figure 4C), and greatest decreases in tumor cell nuclear/cytoplasmic ratio (a morphologic feature of cell differentiation) (Figure 4D), occurred with the THU combined with decitabine or 5-azacytidine over the respective single agents.

Besides direct effects on tumor tissues, we also examined the effects of these treatments on peripheral blood immune-effector and immune-suppressor cell numbers. Peripheral blood CD8+ and CD4+ T-cells were significantly and similarly increased (Figure 10A), while peripheral blood granulocytic and monocytic myeloid-derived suppressor cells (G-MDSC and M-MDSC) were significantly and similarly decreased (~4-fold), by all the treatments vs vehicle (Figure 10A). In tumor tissues, THU-decitabine and THU-5-azacytidine significantly increased infiltration by CD8+ and CD4+ T-cells relative to decitabine or 5-azacytidine alone (Figure 4E). Tumor infiltration by T regulatory cells (T_{reg}) was similarly decreased by all the treatments (Figure 10C), although numbers of peripheral blood regulatory T cells (T_{reg}) did not change significantly (Figure 10B). Suggesting a possible basis for increased T cell infiltration of tumor, expression levels of cancer-germline antigens (Mage-A1, Mage-A3, Mage-A4, Mage-A6) were increased by all the treatments vs vehicle, but most (up to 6-fold) by THU-decitabine or THU-5-azacytidine (Figure 4F, 10D). The antigen presenting molecule MHC class I H-2K^{b}/H-2D^{b} was similarly increased ~2-fold by all treatments (Figure 4G, 10E).

### Alternating decitabine with 5-azacytidine further enhanced efficacy

Decitabine and 5-azacytidine caused downregulations of their respective activity-rate-limiting enzymes, DCK and UCK2, but simultaneously upregulated respectively UCK2 and DCK, an effect we refer to as 'cross-priming' (Figure 2). Thus, in addition to combination with the CDA-inhibitor THU, we alternated decitabine with 5-azacytidine, to avoid the auto-dampening and instead exploit the cross-priming. Mice engrafted with syngeneic LL3 cells were assigned to treatment with (i) vehicle; (ii) THU-decitabine; (iii) THU-5-azacytidine; or (iv) THU-decitabine alternating with THU-5-azacytidine (Figure 5A). Treatment was initiated at Day 21, after confirmation of tumor engraftment by chemi-luminescence (Figure 5B). Repeat imaging on Day 35 demonstrated that the alternating regimen reduced tumor burden the most (Figure 5B), and increased time-to-distress by up to 40 days versus THU-decitabine or THU-5-azacytidine alone (Figure 5C). Analyses of tumor tissue harvested at the time-of-euthanasia suggested the greatest reduction in nuclear/cytoplasmic ratio in H&E-stained tissue sections (Figure 5D), and also greatest Dnmt1-depletion, Myc-downregulation and p27/Cdkn1b upregulation by the alternating regimen (Figure 5E). Besides direct effects on tumor tissues, we examined the effects of these treatments on peripheral blood immune-effector and -suppressor profiles: absolute numbers of peripheral blood CD8+ and CD4+ T-cells were significantly and similarly increased (Figure 11A), while peripheral blood G-MDSC and M-MDSC were significantly and similarly decreased 2-4 fold, by the different treatments versus vehicle (Figure 11A). In tumor tissues, the alternating regimen increased infiltration by CD8+ and CD4+ T-cells over THU-decitabine or THU-5-azacytidine alone (Figure 5F). Although numbers of peripheral blood regulatory T_{reg} were not significantly changed by the treatments vs vehicle, all the treatments decreased T_{reg} by similar amounts in tumor tissue (Figure 11B, C). All three treatment regimens similarly increased expression of cancer-germline antigens up to 12-fold (Mage-A1, Mage-A3, Mage-A4, Mage-A6) (Figure 5G, 11D).

### Addition of anti-Pd1 ICB to THU-decitabine

We evaluated addition of anti-Pd1 ICB to Dnmt1-depletion by THU-decitabine in the model of syngeneic disseminated lung cancer (3-5). After confirmation of tumor engraftment in the lungs using chemi-luminescent imaging on Day 20, mice were distributed to treatment with (i) vehicle; (ii) THU-decitabine alone; (iii) anti-Pd1 alone or (iv) combination THU-decitabine/anti-Pd1 (n = 5/group) (Figure 6A). Both THU-decitabine alone and anti-Pd1 alone decreased tumor burden (measured by chemiluminescence at Day 35) (Figure 6B) and significantly increased time-to-distress versus vehicle by up to 40 days, with a slight advantage for anti-Pd1 alone over THU-decitabine alone (Figure 6D). The combination of anti-Pd1 and THU-decitabine, however, eliminated tumor completely in 2/5 mice (these mice did not become distressed) (Figure 6B). Indicative of an immune-component to this effect, neither of these mice engrafted lung cancer upon tail-vein reinnoculation on Day 90. The mice were sacrificed 40 days after this re-inoculation and careful examination of the lung did not reveal any tumor - while control mice inoculated at the same time succumbed to cancer by day 40 (Figure 6C). Absolute numbers of peripheral blood CD8+ and CD4+ T-cells were significantly and similarly increased (Figure 12A), while peripheral blood G-MDSC and M-MDSC were significantly and similarly decreased (~4-fold), by the different treatments vs vehicle (Figure 12A). Tumor infiltration by CD8+ and CD4+ T-cells was increased the most (~10-fold) by the combination of THU-decitabine and anti-Pd1 (Figure 6E). Peripheral blood T_{reg} were significantly decreased by anti-Pd1 and the anti-Pd1/THU-decitabine combination *vs* the other treatments, and T_{reg} infiltration of tumors was similarly decreased by all three treatments, but not to a significant extent (Figure S4B, C). Dnmt1-depletion, Myc-downregulation and p27/Cdkn1b upregulation was greatest with the combination treatment (Figure 12D). T-cell oligoclonality, defined as the percentage of the T-cell population represented by the 1000 most abundant clones, and measured by RNA-sequencing to determine peripheral blood T-cell receptor (TCR) diversity, was significantly increased in mice cured by the combination treatment compared to vehicle-treated controls (Figure 6F).

### Addition of anti-PD1 ICB to alternating THU-decitabine/THU-5-azacytidine

Since alternating THU-decitabine with THU-5-azacytidine more effectively depleted Dnmt1 from the lung cancer cells in vivo, we evaluated the addition of anti-Pd1 immunotherapy to this epigenetic regimen in the model of syngeneic disseminated lung cancer. After confirmation of tumor engraftment in the lungs using chemi-luminescent imaging on Day 20, mice were distributed to treatment with (i) vehicle; (ii) THU-decitabine/THU-5-azacytidine; (iii) anti-Pd1; or (iv) combination epigenetic/anti-Pd1 therapy (n=5/group) (Figure 7A). Both the epigenetic regimen alone and anti-Pd1 alone decreased tumor burden (measured by chemiluminescence at Day 35) (Figure 7B) and significantly increased time-to-distress vs vehicle by up to 40 days, with a slight advantage for anti-Pd1 alone over the epigenetic therapy alone (Figure 7D). The combination of epigenetic with immunotherapy, however, eliminated tumor completely in 3/5 mice (accordingly, these mice did not become distressed) (Figure 7B-D).

These three mice did not engraft lung cancer upon tail-vein re-challenge with the cancer cells on Day 112. The mice were sacrificed 60 days after this re-inoculation and careful examination of lungs did not reveal any tumor (Figure 7D). Control mice inoculated at the same time succumbed to cancer by day 40 (Figure 7C). Absolute numbers of peripheral blood CD8+ and CD4+ T-cells were significantly and similarly increased (Figure 13). In contrast, absolute numbers of peripheral blood G-MDSC and M-MDSC were decreased by the different treatments vs vehicle (Figure 13). Tumor infiltration by CD8+ and CD4+ T-cells was significantly increased the most (~6-8-fold) by the epigenetic treatment alone or combination epigenetic-immunotherapy vs vehicle (Figure 7E). T-cell oligoclonality as measured by TCR diversity analysis of peripheral blood mononuclear cells using RNA sequencing was significantly increased in mice cured by the combination treatment compared to vehicle-treated controls (Figure 7F). Although peripheral T_{reg} numbers were unchanged by the different treatments vs vehicle (Figure 13C), infiltration of tumor by T_{reg} was significantly and similarly decreased (Figure 13D).

The above comparisons were after different treatment-durations dictated by different times-to-distress of the mice, and complete regression of tumors in some mice limited the ability to analyze treatment-effects on tumor tissue. The experiment was therefore repeated with sacrifice of all mice on the same treatment day 36 (after 14 days of treatment), when vehicle-treated mice became distressed (Figure 14A). Both the epigenetic regimen alone and anti-PD1 alone decreased tumor burden by similar amounts (measured by chemiluminescence at Day 35 tumor weight at Day 36), but combination epigenetic-immunotherapy decreased the tumor burden the most by ~70% (Figure 14B, C). Dnmt1-depletion, Myc-downregulation and p27/Cdkn1b upregulation were also greatest with combination treatment (Figure 15A).

Peripheral blood CD8+ and CD4+ T-cells were significantly and similarly increased (Figure 15B). By contrast, absolute numbers of peripheral blood G-MDSC and M-MDSC were similarly decreased by the different treatments vs vehicle (Figure 15B). Tumor infiltration by CD8+ and CD4+ T-cells was significantly increased by the epigenetic treatment alone or combination epigenetic-immunotherapy vs vehicle (Figure 14D). T_{reg} infiltration of tumor was decreased by all three treatments, but not to a significant extent (Figure 15D). T-cell oligoclonality of tumor infiltrating lymphocytes (TILs) as measured by TCR diversity analysis was significantly increased by the combination treatment compared to vehicle-treated controls (Figure 14E). The different treatments also significantly increased expression of the *Pd1* check-point in TILs (isolated magnetically from tumor tissue) and *Pdl1* gene expression on tumor cells, with the largest increase produced by the combination treatment (Figure 14F). We also measured expression of several other immune checkpoints in the tumor tissue (*Cd80, Cd86, Cd276, Galectin-9, Cd74, Cd155*) and in TILs (*Lag3, Ctla4, Tim3).* Of these, only expression of Cd155 was also consistently increased in tumor tissue with the different treatments vs vehicle (Figure 16).

### Combination ICB and THU-decitabine/THU-5-azacytidine in syngeneic SCLC

We then evaluated the epigenetic-immunotherapy treatment in the syngeneic p53-null SCLC model: F1339-luc SCLC cells were inoculated by tail-vein into syngeneic B6/129 F1 mice. After confirmation of engraftment in liver by live-imaging on Day 13, mice were distributed to treatment with (i) vehicle, (ii) THU-decitabine/THU-5-azacytidine, (iii) anti-Pd1, or (iv) combination epigenetic/immunotherapy (Figure 8A). Anti-Pd1 treatment alone only modestly decreased tumor burden as measured by chemiluminescence imaging on Day 28 (Figure 8B). In contrast, the epigenetic regimen alone and combination epigenetic-immunotherapy significantly and similarly decreased tumor burden (measured by chemiluminescence at Day 28) (Figure 8B) and significantly and similarly increased time-to-distress vs vehicle, with a large advantage over anti-Pd1 alone (Figure 8C). Absolute numbers of peripheral blood while peripheral blood CD8+ and CD4+ T-cells were significantly and similarly increased (Figure 8E), while peripheral blood G-MDSC and M-MDSC were decreased, by the different treatments vs vehicle (Figure 8D).

To compare different treatments administered for the same durations, the experiment was repeated but with sacrifice of all mice after 14 days of treatment (Day 25 when vehicle-treated mice became distressed) (Figure 17A). Anti-Pd1 treatment alone only modestly decreased tumor burden as measured by chemiluminescence imaging (Figure 17B) and by liver weight (Figure 17C). By contrast, the epigenetic regimen alone and combination epigenetic-immunotherapy significantly and similarly decreased tumor burden (Figure 17B, C), with a prominent advantage over anti-Pd1 alone (Figure 17B, C). Tumor tissue histological sections showed decreased nuclear-cytoplasmic ratio and increased necrosis with the epigenetic regimen alone or combination epigenetic-immunotherapy vs vehicle or anti-Pd1 alone (Figure 17D). The epigenetic treatment alone and combination epigenetic-immunotherapy also produced significant increases in antigen-presenting H-2K^{b}/H-2D^{b} molecules (Figure 17E). Oligoclonality of TILs, measured by RNA-sequencing of the T-cell receptor, was also most prominent with epigenetic treatment alone and combination epigenetic-immunotherapy vs vehicle or anti-Pd1 alone (Figure 17F). Absolute numbers of peripheral blood CD8+ and CD4+ T-cells were significantly and similarly increased (Figure 18), while peripheral blood G-MDSC and M-MDSC were decreased, by the different treatments vs vehicle (Figure 18).

To investigate why anti-Pd1 treatment did not add much benefit to the epigenetic treatment in the syngeneic SCLC model (contrasting with the results in the NSCLC models) we measured expression of several immune checkpoint pathway genes in SCLC tumor tissue (*Pdl1, Cd80, Cd86, Cd276, Galectin-9, Cd74, Cd155),* and in TILs isolated magnetically from the tumor tissue (*Pd1, Lag3, Ctla4, Tim3*) after the 14 days of treatment. Treatment of SCLC by anti-Pd1 was linked with several-fold increases in expression of multiple immune-checkpoints other than Pd1/Pdl1, namely Cd74, Cd80, Galectin 9, Tim3, Ctla4 and Lag3 (Figure 19).

The pyrimidine nucleoside analog pro-drugs decitabine and 5-azacytidine traverse pyrimidine metabolism, each by their own path, and convert into the nucleotide Aza-dCTP to deplete the epigenetic target DNMT1. We found here that both pro-drugs induce rapid changes in expression by lung cancer cells of key pyrimidine metabolism enzymes DCK, UCK2, CDA and CAD in ways expected to impede uptake, nucleotide conversion and DNMT1-depletion by the applied agent, but enhance DNMT1-depletion if the sister agent is applied and CDA is inhibited. Specifically, decitabine upregulated UCK2 and CDA ~3-fold within 72-96 hours - UCK2 salvages cytidine, enabling cells to bypass the deoxycytidine analog decitabine. CDA deaminates decitabine into uridine moiety counterparts that do not deplete DNMT1. On the other hand, 5-azacytidine upregulated DCK ~3-fold and CAD ~2-fold - DCK salvages deoxycytidine, enabling cells to bypass the cytidine analog 5-azacytidine; CAD bypasses 5-azacytidine (and decitabine) by generating cytidines de novo. Of these enzyme expression shifts, rapid upregulation of CDA by decitabine, and of DCK by 5-azacytidine, has been reported previously. Decitabine upregulated CDA in leukemia and solid tumor cells by 6 to 1000-fold within 96 hours (30, 31), while 5-azacytidine upregulated DCK in leukemia cells by ~30% within 48 hours (32).

Why do decitabine and 5-azacytidine rapidly and distinctly change pyrimidine metabolism enzyme expression? Decitabine and 5-azacytidine drive levels of dCTP, a key metabolic end-product that regulates nodes in the pyrimidine metabolism network (16), in opposite directions - decitabine increases dCTP and 5-azacytidine decreases it. After phosphorylation by DCK and deamination by DCTD, decitabine inhibits TYMS (~10% of decitabine is expected to traverse this route) - this reduces cellular dTTP by >50% but increases dCTP, because lower dTTP dis-inhibits ribonucleotide reductase to generate more dCTP (ribonucleotide reductase conversion of CDP into dCDP is allosterically controlled by dTTP) (17-19). By contrast, 5-azacytidine *decreases* dCTP by partially inhibiting ribonucleotide reductase (di-phosphorylated 5-azacytidine is a substrate for ribonucleotide reductase) (21). In summary, while the present invention is not limited to any particular mechanism and an understanding of the mechanism is not necessary to understand or practice the invention, it is believed that opposite effects of decitabine vs 5-azacytidine on cellular dCTP amounts (17-19, 21) plausibly drive the diverging adaptive responses of pyrimidine metabolism to the individual pro-drugs. Notably, direct regulation of eukaryotic gene transcription by metabolites has been described (33).

DNMT1 is highly validated scientifically as a molecular target for p53-independent (non-cytotoxic) cytoreduction of both solid tumor and myeloid malignancies, but solid tumor clinical trial results with decitabine or 5-azacytidine have been largely disappointing, especially relative to meaningful results and regulatory approval to treat myeloid malignancies (7). One basis for this discrepancy between myeloid and solid tumor malignancies, that also explains why decitabine and 5-azacytidine are not routinely administered by the oral route, is trivial distribution through high-CDA solid tissues such as the intestines and liver (9, 34). CDA rapidly deaminates both decitabine and 5-azacytidine into non-DNMT1-depleting but potentially cytotoxic uridine nucleoside analogs, shortening in vivo plasma half-lives to ~15 minutes vs 9-16 hours in vitro at 37°C (9, 35-38). Accordingly, addition of the CDA-inhibitor THU increases by ~10-fold the oral bioavailability and plasma half-lives of decitabine and 5-azacytidine, thus enabling tumor cytoreductions even in CDA-rich solid tissue organs (9, 10, 34, 39). CDA upregulation within cancer cells has also been reported by several groups to be a mechanism by which cancer cells resist cytidine analog drugs (reviewed in (9)). Thus, there are both extra-cellular and intra-cellular rationales for combining decitabine or 5-azacytidine with the CDA-inhibitor THU, and THU consistently increased the efficacy of these drugs ~2-fold, without bone marrow suppression, in our in vivo studies of murine lung cancer.

Another significant pharmacologic barrier to decitabine and 5-azacytidine activity in solid vs myeloid malignancies is several-fold lower baseline expression in solid tumors of DCK and UCK2, which as discussed earlier, rate-limit respectively decitabine and 5-azacytidine uptake rates and intra-cellular half-lives (15). Decitabine induction of UCK2, and 5-azacytidine induction of DCK is a practical method to attack this barrier - THU-decitabine alternating with THU-5-azacytidine significantly increased DNMT1-depletion and therapeutic benefit over THU-decitabine or THU-5-azacytidine alone in the murine models of lung cancer, again without causing myelosuppression (the myeloid compartment consists of waves of cells that proliferate transiently then terminally differentiate, likely creating less opportunity for auto-dampening and cross-priming).

DNMT1-depletion has also been extensively shown to increase immune-recognition of cancers by upregulating genes that mediate antigen presentation, type I and type II interferon signaling, and viral defense (3), and hence responses to ICB (40). The pre-clinical evidence is such that there are >10 clinical trials evaluating the combination of parenteral decitabine or 5-azacytidine with ICB (4, 5, 41-43). Unfortunately, the same pharmacologic barriers to previous attempts at clinical translation also threaten these trials. In the present Example, the DNMT1-depleting regimens designed to overcome these barriers produced greater upregulation of antigen presenting MHC class I molecules (MHC I H-2K^{b}/H-2D^{b}) and cancer-germline antigens (MageA1-A4) (that are displayed in MHC class I). Also encouraging, there was greater infiltration of tumor by cytotoxic T-cells and greater shifts towards T cell oligoclonality, and activation of T-cells with increased expression of IFN-γ, perforin and granzyme B. The non-cytotoxic mechanism of action preserved immune-effectors and made possible long-term, chronic therapy, shown also previously in pre-clinical murine models of acute myeloid leukemia (9, 26, 29) and non-human primates (10), and clinically in patients with myeloid malignancies and non-malignant disease treated with non-cytotoxic regimens of parenteral decitabine, oral THU-decitabine or oral THU-5-azacytidine (27, 34, 39, 44). Since clinical data have suggested that reduction of immunosuppressive regulatory immune cells by DNMT1-depleting drugs may be as important as stimulating effector cell-mediated antitumor immunity (45), it is notable that the pharmacologically optimized DNMT1-depleting regimens were also superior to decitabine or 5-azacytidine alone in decreasing numbers of MDSCs and regulatory T-cells. Accordingly, when combined with ICB, complete tumor regressions were produced in some animals, and these animals even resisted fresh tumor cells inoculated by repeat tail-vein injection, a remarkable benefit that implied generation of memory T-cell responses against tumor antigens (46).

Although the non-cytotoxic Dnmt1-depleting regimen significantly cytoreduced p53-null SCLC, increased tumor infiltration by immune-effectors and suppressed MDSC, the addition of ICB using anti-Pd1 did not add much more benefit. While the present invention is not limited to any particular mechanism, one possible explanation is that SCLC suppresses or 'checks' immune attack using as dominant pathways molecules other than Pdl1. Several immune checkpoints other than Pd1 were significantly upregulated in the SCLC tumors treated with anti-Pd1, including the Tim3, Ctla-4 or Lag3 checkpoint pathways.

Scientific rationale for decitabine and 5-azacytidine combinations with immune-therapies is compelling enough that >10 solid tumor clinical trials are underway or completed, despite disappointing results from preceding trials (5, 7). We propose that persistent translation difficulties could reflect pharmacology problems, such as disadvantageous baseline expression patterns of the key pyrimidine metabolism enzymes CDA, DCK and UCK2 in solid tumors such as lung cancer, that are further exacerbated by adaptive shifts in expression in response to decitabine or 5-azacytidine.

### MATERIALS AND METHODS

### Study design

The objectives of this study were to identify potential mechanisms underlying failure of clinical trials combining DNMT1-depleting pyrimidine nucleoside analog pro-drugs with ICB, and to evaluate mechanism-based solutions. The central hypothesis was that the regulated network structure of pyrimidine metabolism reacts to automatically dampen activity of administered pyrimidine nucleoside analogs, and that these adaptive responses of metabolism can be anticipated and harnessed to improve response instead. The experimental approach first validated the key pyrimidine metabolism enzymes mediating nucleoside analog pro-drug activity, by correlating in vitro drug-sensitivity (growth-inhibition) in the NCI60 panel of cancer cell lines with expression of pyrimidine metabolism enzymes. Then, how expression of key pyrimidine metabolism enzymes changes upon cancer cell exposure to nucleoside analogs versus natural pyrimidine nucleosides was determined using at least three independent biological replicates. Two in vivo models of aggressive, disseminated syngeneic murine lung cancer were then used to evaluate candidate solutions to exploit adaptive responses of the pyrimidine metabolism network, both alone and in combination with ICB. Mice cured by combination epigenetic-immunotherapy were re-challenged by tail-vein inoculation of cancer cells to evaluate if the mechanism of cure incorporated immune memory against the cancer. Live imaging was used to verify tumor engraftment prior to initiation of therapy, with mice distributed to treatment groups to balance baseline tumor burden. Each in vivo experiment was powered to show statistically significant results between treatment groups, possible with five mice per treatment group because of treatment-effect sizes. Eight independent in vivo treatment experiments were conducted, with >180 mice treated in total. All mice were female, to avoid confounding of interpretation by sex-differences in pro-drug metabolism. All in vivo experiments validated that DNMT1-depleting therapy preserved immune-effectors and increased tumor immune-recognition/infiltration, using standard methods. That non-cytotoxic DNMT1-depletion can cytoreduce chemorefractory p53-null cancer was verified both in vitro and in vivo.

### Cell lines and culture

LL3 Lewis lung cancer cells were employed. F1339 small cell lung cancer cells were also employed. Both cell lines were maintained in RPMI1640 supplemented with 10% fetal bovine serum (FBS, Gibco), 100 units/ml penicillin, 100 µg/ml streptomycin (Gibco), and cultured at 37°C in 5% CO₂. These cell lines are not in the database of commonly misidentified cell lines (ICLAC and NCBI Biosample). The cell lines regularly tested negative for *Mycoplasma* contamination.

### Mice

Female C57BL/6, B6/129SF1/J and NSG mice were purchased from Jackson Lab (Bar Harbor, ME, USA). Mice were inoculated with tumor cells at age 4-6 week old. Mice were maintained under specific pathogen-free conditions, with free access to food and water. All procedures were performed in compliance with the legislation on the use and care of laboratory animals, and according to protocols (2013-1137 and 2017-1863) approved by the Institutional Animal Care and Use Committee (IACUC) of Cleveland Clinic.

### Syngeneic tumor models

C57BL/6 mice were tail-vein inoculated with 0.35×10⁶ LL3 cells, and B6/129SF1/J mice with 0.3×10⁶ F1339 cells. Assignment to different treatments was after documentation of tumor engraftment by live imaging. Peripheral blood monitoring on-treatment was by tail-vein phlebotomy. Mice were euthanized for signs of distress as defined in the Animal Protocols, and blood and tumor were collected for further analyses. Apparently cured mice were re-challenged with fresh tumor cells (0.5×10⁶) inoculated by tail vein.

### Patient-derived xenotransplant model

Primary tumor and pleural fluid was collected from a lung cancer patient after written informed consent on IRB-approved protocol (07-267). Six-week-old female NSG mice were inoculated in the right flank with 5×10⁶ tumor cells. On day 3 after tumor inoculation, 5×10⁶ human PBMCs from the same patient were inoculated via tail-vein. Mice were distributed such that baseline tumor volume was similar between treatment groups. Tumor volume was recorded twice a week and the experiment was terminated when the tumor volume in vehicle treated mice reached 2000 mm³.

### Flow cytometry analysis

Peripheral blood mononuclear cells were stained with CD3, CD4, CD8, CD11b, Ly6C, Ly6G antibodies (BD Biosciences). The stained cells were then fixed with fixation buffer (eBioscience). The FOXP3 staining buffer set (eBioscience) was used for intranuclear staining. For flow cytometry analyses of tumor tissue, tumor tissue was first digested by cutting into small fragments then incubated with mouse or human tumor enzyme cocktail per the manufacturer's protocol with gentlMACS tubes and the gentleMACS dissociator (Miltenyi). After filtering through a 70-µm strainer (Thermo Fisher Scientific), the single-cell tumor suspension was centrifuged on a 30% percoll gradient over a 70% percoll gradient to enrich for mononuclear cells and remove debris. Cells were stimulated with PMA/ionomyocin in the presence of Golgi stop and Monesin (eBioscience) for 4 hours, then washed with PBS and stained with Live/Dead stain (Life Technologies) and antibodies specific to cell surface markers CD3, CD4 and CD8 (BioLegend). After fixation-permeabilization (Fixation/Permeabilization buffer, eBioscience), cells were stained with antibodies specific to IFNr, FOXP3, granzyme B and perforin (BioLegend) or with the isotype control antibodies. Flow cytometry analysis was performed on BD LSRFortessa and data was analyzed by FlowJo V10.

### Quantitative polymerase chain reaction

RNA was extracted using Trizol reagent (Invitrogen) before the generation of complementary DNA using the iScript^{™} cDNA Synthesis Kit (Bio-rad). Quantitative PCR was run on StepOnePlus Real-Time PCR System (Applied Biosystems), using comparative *C*ₜ value method to quantify the expression of target genes in different samples. Gene expression was normalized to the housekeeping gene *β-actin.*

### Western blot

Tumor cells were lysed with RIPA lysis buffer and protein concentrations determined by BCA protein assay kit (Thermo Fisher Scientific). Samples with equal quantity (40 µg) of total protein were mixed with 4× loading buffer and 10× sample reducing reagent (Thermo Fisher Scientific), subjected to electrophoresis on a 12% (v/v) SDS-polyacrylamide gel, then transferred onto polyvinylidene fluoride membranes. After blocking with 5% dried skimmed milk, the membranes were washed three times and incubated with primary antibodies at 4 °C overnight. After washing, the membranes were further incubated with corresponding horseradish peroxidase-conjugated secondary antibodies. The membranes were then treated with PierceTM ECL substrates (Thermo Scientific) then visualized using X-ray film.

### Histological analysis

Tumors were fixed in 10% buffered formalin phosphate (Thermo Fisher Scientific) for 12 hours and embedded in paraffin. Sections were stained using H&E.

### T-cell receptor (TCR) sequencing (T-cell oligoclonality analyses)

RNA was isolated from PBMC or CD8+ TILs using the AllPrep RNA Mini Kit (Qiagen) - 200 ng of total RNA was used to construct TCR alpha and beta chain (a/b) libraries using the SMARTer Mouse TCR a/b Profiling Kit (Takara) per manufacturer's instruction. Samples were pooled to a final concentration of 4 nM and then the pooled libraries were further diluted to a final concentration of 13.5 pM including a 7% PhiX Control v3 (Illumina) spike-in. Sequencing was performed on an Illumina MiSeq sequencer (Illumina) using the 600-cycle MiSeq Reagent Kit V3 (Illumina) with paired-end, 2 × 300 base pair reads. The data was analyzed with MiXCR 1.1.0 (Illumina).

### Statistical analysis

Statistical analysis was performed with Prism 7.0 software (GraphPad, San Diego, CA). Survival differences among the treatment groups were analyzed by the Kaplan-Meier method and *p* values were calculated with log-rank test. Two-sided Mann-Whitney *U* test was used to compare medians and a two-sided unpaired t test to compare means. Bonferroni's correction to p<0.05 was used to determine statistical significance.

### EXAMPLE 2

### Human Cancer Treatment with Staggered Decitabine and 5-Azacytidine

This Example describes various treatment protocols that could be used to treat human patients with cancer, such as pancreatic cancer.

Figure 20 describes a first exemplary treatment staggered decitabine and 5-azacytidine weekly treatment protocol for a human patient with cancer over a treatment period of multiple weeks. This protocol includes the possibility of skipping treatment a given week if absolute neutrophil count (ANC) is too low. In such instances, the patient can be administered G-CSF.

Figure 21 describes a second exemplary treatment staggered decitabine and 5-azacytidine weekly treatment protocol for a human patient with cancer over a treatment period of multiple weeks. In this protocol, there is an induction period where the patient is administered THU and then decitabine 60 minutes later for five consecutive days, and then G-CSF (NEULASTA neutropenia prophylaxis) anytime between days 5-9.

Figure 22 describes a third exemplary treatment staggered decitabine and 5-azacytidine weekly treatment protocol for a human patient with cancer over a treatment period of multiple weeks. This protocol has a first induction protocol and potential second induction protocol as described in Figure 22.

A fourth exemplary protocol is as follows. THU-Decitabine administered to a human with cancer on day 1, THU-5-Azactidiine administered on day 4, of every week for at least four weeks. All drugs are oral. THU dose is ~10 mg/kg; capsules are 250 mg each. Decitabine dose is ~0.16 mg/kg; capsules are 5 mg each. 5-Azacitidine dose is 1.6 mg/kg; capsules are 50 mg each. The following dosing schedule is used for starting doses: i) weight ≤45 kg: 2 capsules of THU followed 60-360 minutes later by 1 capsule of decitabine (on Day 1) or 1 capsule of 5-azacytidine (on Day 4) (e.g., THU at breakfast, Decitabine or 5-azacytidine at lunch); ii) weight 45-80 kg: 3 capsules of THU followed 60-360 minutes later by 2 capsules of decitabine (on Day 1) or 2 capsules of 5-azacytidine (on Day 4); and iii) weight 81 kg or higher: 4 capsules of THU followed 60-360 minutes later by 3 capsules of decitabine (on Day 1) or 3 capsules of 5-azacytidine (on Day 4).

### EXAMPLE 3

### Timed Decitabine and 5-Azacytidine Treatment

### METHODS

**Study approvals.** Bone marrow samples for research were obtained from patients with AML (Acute myeloid leukemia) with written informed consent on a study protocol approved by the Cleveland Clinic Institutional Review Board (Cleveland, Ohio). Murine experiments were approved by the Cleveland Clinic Institutional Animal Care and Use Committee (Cleveland, Ohio).

***Sources of cell lines and animals.*** AML cell lines OCI-AML3 were purchased from DSMZ (Braunschweig, Germany), and THP1, K562 and MOLM13 cell lines were purchased from ATCC (Manassas, Virginia). The AML cell lines, including those selected for resistance to decitabine, were authenticated (Genetica cell line testing, Burlington, NC). *DCK* and *UCK2* knock-out leukemia (HAP1) cells were engineered via Horizon Discoveries (Cambridge, United Kingdom). Primary AML cells for inoculation into NSG mice were collected with written informed consent on Cleveland Clinic Institutional Review Board approved protocol 5024. NSG mice were purchased from Jackson Laboratories (Bar Harbor, Maine).

***DNMT1 Immuno-detection and quantitation:*** Immunohistochemistry (IHC) was performed on decalcified and formalin-fixed paraffin embedded bone marrow biopsy sections (4µm) and on positive and negative controls (parental and DNMT1-KO HCT116 cells). Antibodies used were mouse polyclonal anti-Dnmt1 (Abcam #ab19905, Cambridge, MA), 1:200 dilution for 32 minutes at room temperature, performed with Ventana Discovery using OmniMap detection and a high pH tris-based buffer (Cell Conditioning 1, Ventana #950-124). Nuclei positive for DNMT1 were identified and quantified in high resolution, large field-of-view images per ImageIQ algorithms (Image IQ Inc., Cleveland, OH) after segmentation of images and subtraction of bone as has been previously described⁴.

DNMT1-protein measurement by flow cytometry was performed as previously described⁴⁰ using unlabeled anti-Dnmt1 antibody [EPR 3522] (0.0625 µg/test; Abcam; catalog no. ab92314) as the primary antibody

***DNA isolation, reverse transcription (RT) and real-time PCR.*** As previously described ⁵⁹. Primer sequences were:

| **Gene** | **Primer (Forward)** | **Primer (Reverse)** |
|---|---|---|
| CAD | 5'-CTGACTTCTACACTGAGCATGG-3' SEQ ID NO:1 | 5'-CACGCATTGACAGGTTAATCAC-3' SEQ ID NO:2 |
| CDA | 5'-AAGGGTACAAGGATTTCAGGG-3' SEQ ID NO:3 | 5'-ACAATATACGTACCATCCGGC-3' SEQ ID NO:4 |
| DCK | 5'-AAGCTGCCCGTCTTTCTC-3' SEQ ID NO:5 | 5'-ACCACTTCCCAATCTTCACAC-3' SEQ ID NO:6 |
| UCK2 | 5'-ATCCCCGTGTATGACTTTGTC-3' SEQ ID NO:7 | 5'-CTTCATCTGGAACAGGTCTCG-3' SEQ ID NO:8 |
| GAPDH | 5'-ACATCGCTCAGACACCATG-3' SEQ ID NO:9 | 5'-TGTAGTTGAGGTCAATGAAGGG-3' SEQ ID NO:10 |

**1D SDS-polyacrylamide gel electrophoresis and Western blot analysis.** Were performed as we previously described ⁵⁹. Antibodies used were:

| **Primary Antibodies Used** | | |
|---|---|---|
| **Catalogue#** | **Name** | **Company** |
| ab13537 | DNMT1 antibody | Abcam |
| sc-393098 | dCK Antibody (H-5), Mouse | Santa Cruz |
| ab104731 | Anti-UCK2 antibody, Rabbit | ABCAM |
| 12662 | Phospho-CAD (Ser1859) | Cell Signaling |
| 11933 | CAD Antibody | Cell Signaling |
| GTX108663 | DCTD antibody | GeneTex |
| sc-390945 | TYMS Antibody (C-5) | Santa Cruz |
| sc-377415 | RRM1 Antibody (A-10) | Santa Cruz |
| sc-398294 | RRM2A Antibody (A-5) | Santa Cruz |
| PRS2383-100UG | Anti-P53R2 antibody (RRM2A) | Sigma-Aldrich |
| ab82347 | Anti-CDA antibody | ABCAM |
| sc-374015 AF488 | Lamin B1 Antibody (B-10) Alexa Fluor^{®} 488 | SCBT |
| sc-47724 AF647 | GAPDH Antibody (0411) Alexa Fluor^{®} 647 | SCBT |
| F3022-.2mL | Actin-FITC | Sigma-Aldrich |
| A304-543A | Rabbit anti-CTPS1 Antibody | Bethyl Lab |

| **Secondary Antibodies Used** | | |
|---|---|---|
| Catalogue# | **Name** | **Company** |
| A32735 | Goat anti-Rabbit IgG (H+L) Highly Cross-Adsorbed Secondary Antibody, Alexa Fluor Plus 800 | Invitrogen |
| A32730 | Goat anti-Mouse IgG (H+L) Highly Cross-Adsorbed Secondary Antibody, Alexa Fluor Plus 800 | Invitrogen |
| 12004158 | StarBright^{™} Blue 700 Goat Anti-Mouse IgG, 400 µl | Biorad |
| 12004161 | StarBright^{™} Blue 700 Goat Anti-Rabbit IgG, 400 µl | Biorad |

Fluorescent images were collected using Biorad's ChemiDoc system and processed with Image lab.

***Giemsa staining of cells.*** As previously described⁵⁹.

***Flow Cytometry Analyses for human and murine CD45.*** As previously described^{59, 60}. Antibodies used were monoclonal anti-human CD45 (clone HI30, cat. No 304016, Biolegend, 1:100) and monoclonal anti-mouse CD45 (Clone 30-F11, cat. No 1031066, Biolegend, 1:100).

***Preparation and analysis of dNTP and NTP extracts:*** Cells were washed twice with ice-cold 1X PBS, and counted. To lyse cells, precipitate proteins and extract nucleotides and nucleosides, for each 5-10 million cells, 250 µL of 80% acetonitrile/water was added, and the cells were incubated on ice for 15min. After incubation, the suspension was centrifuged at 140K rpm for 5min. The supernatant from this first extraction was transferred to a clean tube. The remaining pellet was extracted again with fresh 80% acetonitrile/water, and supernatant from both extractions was combined, and then evaporated to dryness using a centrifugal evaporator. *LCMS*/*MS:* 1mM Internal standards (13C9 15N3MP and d 13C9 15N3TP) solution (25mM ammonia acetate, 10mM DMHA, pH 8.0) was used to suspend Nucleotides and nucleosides extract. HPLC separation was carried on an ACQUITY UPLC HSS T3 Column, 100Å, 1.8 µm, 2.1 mm X 150 mm. A stepwise gradient program was applied with mobile phase A (25mM Ammonia Bicarbonate, 10mM DMHA, pH 8.0) and mobile phase B (60% Acetonitrile/water). The HPLC was interfaced with Thermofisher Quantiva triple quadruple mass spectrometer. The mass spectrometer was operated in MRM mode with optimized MRM transitions for each analyte. *Data analysis:* Xcalibur was used to process and quantify raw data. Briefly, a processing method was built using MRM transitions and peak retention times from standards. All samples were processed with the same method to generate integrated total ion intensity (integrated peak area) for each analyte. Manual inspection was performed to confirm the peak assignment and integration. The final report value was normalized to the internal standards and total number of cells used to generate the extract.

***Treatment of a patient-derived xenotransplant model of treatment-resistant AML.*** Patient-derived primary AML cells from a patient with AML that had progressed on standard chemotherapy then decitabine salvage therapy, were transplanted by tail-vein injection (1.0 x10⁶/mouse) into non-irradiated 6-8 week old NSG mice. Mice were anesthetized with isofluorane before transplantation. Mice were randomized to different treatments on Day 9 after inoculation, with treatments as indicated in each figure and legend. Doses of drugs used were: intra-peritoneal tetrahydrouridine (THU) 10 mg/kg given intra-peritoneal up to 3X/week; subcutaneous decitabine 0.2 mg/kg up to 3X/week (or 0.1 mg/kg when combined with THU); subcutaneous 5-azacytidine 2 mg/kg up to 3X/week (or 1 mg/kg when combined with THU); intra-peritoneal dT 2 g/kg up to 2X/week. Tail-vein blood samples for blood count measurement by HemaVet were obtained prior to leukemia inoculation, and at intervals thereafter as indicated in the figures. Mice were observed daily for signs of pain or distress, e.g., weight loss that exceeded 20% of initial total body weight, lethargy, vocalization, loss of motor function to any of their limbs, and were euthanized by an IACUC approved protocol if such signs were noted.

*Bioinformatic and statistical analysis.* Wilcoxon rank sum, Mann Whitney, and t tests were 2-sided unless otherwise stated because of apriori literature-based hypotheses (dCTP level analyses) and performed at the 0.05 significance level or lower (Bonferroni corrections were applied for instances of multiple parallel testing). Standard deviations (SD) and inter-quartile ranges (IQR) for each set of measurements were calculated and represented as y-axis error bars on each graph. Graph Prism (GraphPad, San Diego, CA) or SAS statistical software (SAS Institute Inc., Cary, NC) was used to perform statistical analysis including correlation analyses.

### RESULTS

### DNMT1 is not depleted at clinical relapse or with in vitro resistance

Serial bone marrow biopsies from the same patient, before and during therapy with decitabine or 5-azacytidine, were cut onto the same glass slide and stained simultaneously to facilitate time-course comparison of DNMT1 protein levels quantified by immunohistochemistry and ImageIQ imaging/software (39 serial bone marrow samples from 13 patients, median treatment duration 372 days, range 170-1391) **(****Figure 23A****, B).** At time-of-response, DNMT1 protein was markedly and significantly decreased by ~50% compared to pre-treatment **(****Figure 23B****).** At the time-of-relapse on-therapy, however, DNMT1 protein levels had rebounded to levels comparable to pre-treatment levels **(****Figure 23B****).**

Since the pyrimidine metabolism enzymes DCK, UCK2, CDA and CAD are well-documented to mediate DNMT1-depleting ability of decitabine and 5-azacytidine **(****Figure 23A****),** we used quantitative polymerase chain reaction (QRT-PCR) to measure expression of these enzymes in MDS patients' bone marrows pre-treatment and at relapse on-therapy with decitabine (n=13, median treatment-duration 175 days, range 97-922) or 5-azacytidine (n=14, median treatment-duration 433 days, range 61-1155) **(****Figure 23C****).** DCK expression decreased by ~50% at relapse on decitabine but increased by ~8-fold at relapse on 5-azacytidine **(****Figure 23C****).** Conversely, UCK2 expression increased by ~8-fold at relapse on decitabine but decreased by ~50% at relapse on 5-azacytidine **(****Figure 23C****).** CDA expression increased ~3-fold at relapse on decitabine and decreased by ~50% at relapse on 5-azacytidine **(****Figure 23C****).** CAD increased an average of ~8-fold at relapse on either pro-drug, but not in all patients **(****Figure 23C****).** The proliferation marker MKI67 increased at relapse in almost all the patients, consistent with active progression of disease **(****Figure 23C****).**

We then evaluated resistance to decitabine in vitro: AML cells (THP1, K562, OCI-AML3, MOLM13) were cultured in the presence of decitabine 0.5 - 1.5 µM (clinically relevant concentrations). After initial cytoreduction, AML cells proliferating exponentially through the decitabine emerged as early as 40 days after the first decitabine addition **(****Figure 23D****, 29).** DNMT1 was not depleted from these decitabine-resistant AML cells despite the presence of decitabine **(****Figure 23D****).** UCK2 and CDA protein levels were markedly elevated in decitabine-resistant vs vehicle-treated parental AML cells **(****Figure 23D****).** Also upregulated was CAD, by total protein levels and by S 1856 phosphorylation, a post-translational modification linked with its functional activation **(****Figure 23D****).** In contrast, DCK protein levels were suppressed **(****Figure 23D****).** In short, the in vitro resistance resembled the clinical resistance, with preserved DNMT1 and consistent pyrimidine metabolism enzyme expression changes.

### Decitabine and 5-azacytidine cause deoxynucleotide imbalances and metabolic compensations

We examined whether decitabine and 5-azacytidine cause deoxynucleotide imbalances, to potentially trigger compensatory responses from the pyrimidine metabolism network_ENREF_54. AML cells (MOLM13, OCI-AML3, THP1) were treated with a single dose of vehicle, natural deoxycytidine 0.5 µM, decitabine 0.5 µM, natural cytidine 5 µM, or 5-azacytidine 5 µM in vitro, and effects on nucleotide levels and pyrimidine metabolism gene expression were measured 24 to 72 hours later **(****Figure 24A****).**

Vehicle, deoxycytidine and cytidine did not impact proliferation of the AML cells **(****Figure 24B****).** A single treatment with either decitabine or 5-azacytidine, on the other hand, significantly decreased AML cell proliferation **(****Figure 24B****).** We then measured impact on dCTP and dTTP amounts at the 24 hour time-point: dCTP was significantly increased by decitabine but significantly decreased by 5-azacytidine **(****Figure 24C****).** dTTP was decreased by both pro-drugs, but not to statistical significance **(****Figure 24C****).** We then measured expression of key pyrimidine metabolism enzymes. Serial Western blot measurements of enzyme protein levels indicated peak expression changes occurred 48-96 hours after addition of pro-drug **(****Figure 30****).** We thus focused repeat measurements, using both QRT-PCR and Western blots, at the 72 hour time-point. DCK mRNA and protein expression was significantly increased by 5-azacytidine but not decitabine treatment **(****Figure 24D****, E, 30).** Conversely, UCK2 mRNA and protein expression was significantly increased by decitabine but not 5-azacytidine **(Figure 2D,** **E,** **30).** CDA mRNA and protein expression was significantly increased by both pro-drugs **(****Figure 24D****, E, 30).** Neither pro-drug changed total CAD or cytidine triphosphate synthetase 1 (CTPS1) levels (CTPS1 executes a late step in de novo pyrimidine **synthesis)(****Figure 24D****, E, 30).** Both pro-drugs did, however, decrease phosphorylation of CAD at serine 1856 (S**1856)(****Figure 24E****)** - CAD S1856 phosphorylation is linked with functional upregulation of CAD-mediated de novo pyrimidine synthesis - thus, the pro-drugs acutely downregulated CAD function. Both decitabine and 5-azacytidine depleted DNMT1 as expected **(****Figure 24E****, 30).**

We extended protein level analyses to additional pyrimidine metabolism enzymes playing nodal roles in nucleotide balance: thymidylate synthase (TYMS) is the major mediator of deoxythymidine triphosphate (dTTP) production. TYMS was downregulated by both pro-drugs, but to a noticeably greater extent by decitabine than 5-azacytidine **(****Figure 24E****, 30) -** the natural substrate of TYMS is deoxyuridine monophosphate (dUMP), and decitabine and 5-azacytidine are metabolized into a dUMP analog Aza-dUMP by 2 *vs* 6 catalytic steps respectively **(****Figure 30****).** 5-azacytidine, but not decitabine, decreased levels of the ribonucleotide reductase sub-unit RRM1 **(****Figure 24E****)** (ribonucleotide reductase converts RNA molecules such as 5-azacytidine, after diphosphorylation, into DNA molecules such as decitabine), with less impact on the ribonucleotide reductase sub-unit RRM2A **(****Figure 30****).**

### DCK and UCK2 are important for maintaining dCTP and dTTP respectively

To better understand contributions of DCK and UCK2 to dCTP and dTTP maintenance, we knocked DCK and UCK2 out of leukemia cells (HAP1) using CRISPR-Cas9 then measured levels of these nucleotides. *DCK*-knockout, but not *UCK2*-knockout, significantly decreased dCTP **(****Figure 25A****, B).** Thus, DCK appears important to dCTP maintenance, consistent with DCK upregulation as an appropriate compensatory response to dCTP suppression by 5-azacytidine **(****Figure 24****).** *UCK2*-knockout, but not *DCK*-knockout, significantly decreased dTTP **(****Figure 25A****, B).** Thus, UCK2 appears important to dTTP maintenance, consistent with UCK2 upregulation as a response to dTTP suppression by decitabine **(****Figure 24****).**

We also examined sensitivity of the *DCK-* and *UCK2*-knockout cells to decitabine and 5-azacytidine. *DCK*-knockout cells were relatively resistant to decitabine (concentrations for 50% growth inhibition [GI50] 12 *vs* 3 µM for parental cells), but more sensitive to 5-azacytidine (GI50 2 *vs* 4 µM for parental **cells)(****Figure 25C****).** *UCK2*-knockout cells were relatively resistant to 5-azacytidine (GI50 15 µM *vs* 4 µM for parental cells), but more sensitive to decitabine (GI50 0.1 µM vs 3 µM for parental **cells)(****Figure 25C****).**

### Resistance countermeasures evaluated in vivo

We then examined solutions to resistance in a patient-derived xenotransplant (PDX) model of AML, derived from a patient with AML that was chemorefractory to both decitabine and cytarabine:
(a) *Schedule decitabine administration to avoid DCK troughs:* Immune-deficient mice were tail-vein innoculated with 1 million of these human AML cells each. On Day 9 after innoculation, mice were randomized to treatment with **(i)** vehicle; **(ii)** decitabine timed to avoid DCK troughs (Day 1 and Day 2 each week - Day 1, 2); or **(iii)** decitabine timed to coincide with DCK troughs (Day 1 and Day 4 each week - Day 1, 4) **(****Figure 31A****).** Vehicle-treated mice showed distress on Day 45, at which point all mice were euthanized or sacrificed for analyses. The bone marrows of vehicle and Day 1, 4 treated mice, but not Day 1, 2 treated mice, were replaced by AML cells observed by light microscope **(****Figure 31B****)** and by flow cytometry - human CD45+ (hCD45+) AML cells were ~92% with PBS, ~63% with Day1, 4 and ~26% with Day 1, 2 treatment **(****Figure 31C****).** Spleens were enlarged with effaced histology by AML with vehicle or Day 1, 4 treatment but had mostly preserved histology with Day 1, 2 treatment **(****Figure 31D****, E).** Spleen weights as another measure of AML burden were also lowest with Day1, 2 treatment **(****Figure 31F****).**

These two schedules of decitabine administration were compared again but with waiting for signs of distress in individual mice rather than collective sacrifice at day 45 **(****Figure 32A****).** Median survival (time-to-distress) was significantly better with Day 1, 2 (75 days) vs Day 1, 4 (60 days) or vehicle treatment (40 days) **(****Figure 32B****),** and bone marrow and spleen AML burden was again lowest with Day 1, 2 *vs* Day 1, 4 or vehicle treatment **(****Figure 32C-E****).**

Thus, scheduling decitabine administration to avoid DCK troughs (Day 1, 2) was superior to scheduling that coincided with these troughs (Day 1, 4).

(b) *Combine with CDA and*/*or ribonucleotide reductase inhibitors:* CDA can be inhibited by THU, while de novo pyrimidine synthesis can be inhibited at ribonucleotide reductase using deoxythymidine (dT) or hydroxyurea. NSG mice tail-vein innoculated with 1 million AML cells each were randomised to **(i)** vehicle; **(ii)** THU+dT, **(iii)** decitabine; **(iv)** THU+decitabine; or **(v)** THU+dT+decitabine **(****Figure 26A****).** PBS and THU/dT-treated mice developed signs of distress, and were euthanized, on day 42. Mice receiving other treatments were sacrificed 3 weeks later (day 63) to increase chances of seeing differences in AML burden between these treatments **(****Figure 26A****).** Visual inspection and flow cytometry demonstrated bone marrow replacement by human AML cells with vehicle and THU+dT treatment (>90% hCD45+), improved by decitabine alone (~85% hCD45+) but most by THU+decitabine (~35% hCD45+) and THU+dT+decitabine (~42% **hCD45+)(****Figure 26BC** )(that is, dT did not add further to the benefit from THU). Murine hematopoiesis was completely suppressed with vehicle and THU+dT (0% murine Cd45+), almost completely suppressed with decitabine-alone (~5% mCd45+) but preserved with THU+decitabine (~40% Cd45+) and THU+dT+decitabine (~27% **Cd45+)(****Figure 26C****).** Hemoglobin and platelets were most suppressed, and white cells (peripheral leukemia) most elevated, with vehicle or THU+dT treatment, but only mildly to moderately suppressed with any of the decitabine containing regimens **(****Figure 26D****).** Spleen weights as a measure of AML burden were lowest with THU+decitabine- and THU+dT+decitabine **(****Figure 26E****).** Spleen histology confirmed replacement by AML cells (with necrotic areas) with vehicle or THU+dT **(****Figure 26F****),** decreased with decitabine-alone, and normal-appearance with THU+decitabine and THU+dT+decitabine **(****Figure 26F****).** We also evaluated the use of hydroxyurea to inhibit ribonucleotide reductase: hydroxyurea 100 mg/kg IP was administered on Day 1 before THU+decitabine on days 2 and 3: hydroxyurea, like dT, did not add further benefit to THU+decitabine **(****Figure 33A****, B).**

(c) *Frequent, distributed vs pulse-cycled schedules of administration:* DNMT1-depletion by decitabine or 5-azacytidine is S-phase dependent, suggesting frequent, distributed administration, to increase chances of overlap between malignant S-phase entries and drug exposures, could be better than pulse-cycled administration over a few consecutive days followed by weeks without therapy, designed for anti-metabolite/cytotoxic therapy that requires long treatment gaps to recover from toxic side-effects. Bone marrow AML burden was lowest with frequent-distributed administration of THU/decitabine 2X/week vs pulse-cycled administration for 5 days every 4 weeks **(****Figure 33C****).** Vehicle-treated mice showed distress on Day 45, at which point all mice were euthanized or sacrificed for analyses. The bone marrows of vehicle treated mice demonstrated ~95% replacement by human CD45+ AML cells. Bone marrow AML burden was decreased to ~60% by pulse-cycled decitabine but decreased most to ~10% with frequent-distributed decitabine, with inversely corresponding murine Cd45+ cells **(****Figure 33D****).**

*(d) Exploit cross-priming by Dec and 5Aza for each other:* We compared head-to-head THU+decitabine 3X/week vs THU+5-azacytidine 3X/week and found no differences in efficacy between these two treatments **(****Figure 27****, 34).** Then, since decitabine appears to cross-prime for 5-azacytidine activity by upregulating UCK2, while 5-azacytidine cross-primes for decitabine activity by upregulating DCK **(****Figure 23-25****, 30),** we alternated THU+decitabine with THU+5-azacytidine week-to-week, and compared this to THU+decitabine or decitabine alone. Mice tail-vein innoculated with patient-derived AML cells (1×10⁶cells/mouse) were randomized to **(i)** vehicle; **(ii)** decitabine alone; **(iii)** THU+decitabine; or **(iv)** THU+decitabine alternating with THU+5-azacytidine week-to-week **(****Figure 28A****).** Median survival (time-to-distress) was best with THU+decitabine/THU+5-azacytidine (221 days) *vs* THU+decitabine (180 days), decitabine-alone (111 days) or vehicle (50 days) **(****Figure 28B****).** Blood count stability during the weekly treatments was consistent with a non-cytotoxic mechanism-of-action of the therapies (shown also previously^{3, 26, 38-} **⁴¹)(****Figure 28C****).** Eventual declines in hemoglobin and platelets were caused by progressive leukemia, shown by increasing peripheral leukemia cells (increasing white cell counts) (Figure **28C****),** and by flow cytometry analyses of bone marrows harvested after euthanasia (Figure **28D****).** Alternating THU+decitabine with THU+5-azacytidine in 4 week cycles, or simultaneous administration of THU+decitabine+5-azacytidine, did not add benefit over THU+decitabine or THU+5-azacytidine alone **(****Figure 27****, 34).** Thus, the benefit of alternating THU+decitabine with THU+5-azacytidine depended on timing of alternation.

### Mechanisms-of-resistance in mice

Bone marrow cells harvested at day 63 when leukemia-inoculated mice were doing well on-therapy demonstrated DNMT1-depletion, with the greatest DNMT1-depletion with THU+decitabine alternating with THU+5-azacytidine week-to-week (~65% DNMT1-depletion) vs THU+decitabine (~50%), decitabine alone (~35%) or vehicle (~15%) **(****Figure 28E****).** By contrast, bone marrow AML cells harvested at the time of progressive leukemia on these therapies demonstrated failure to deplete DNMT1 as measured by flow-cytometry **(****Figure 28E****).** These bone marrows also demonstrated significant upregulations of CDA and CAD, with the greatest upregulations in AML cells from mice that received the alternating regimen and survived the longest **(****Figure 28F****).**

Unbiased pre-clinical genetic studies have verified the central roles of DCK and UCK2 in determining sensitivity of leukemia cells to the pro-drugs decitabine and 5-azacytidine (companion manuscript). Here we found that malignant myeloid cells in vitro, in mice and in patients avoided DNMT1-depletion and resisted decitabine or 5-azacytidine via changes in expression of these and other key pyrimidine metabolism enzymes. We moreover found that these enzyme expression changes emerged from adaptive responses of the pyrimidine metabolism network, that senses and regulates deoxynucleotide amounts - decitabine and 5-azacytidine had opposite effects on dCTP amounts, via off-target depletion of TYMS (the major mediator of cellular dTTP production) and RRM1 (a key sub-unit of ribonucleotide reductase, the enzyme complex that converts ribonucleosides such as 5-azacytidine, after their diphosphorylation, into deoxyribonucleosides) respectively. TYMS, like DNMT1, methylates carbon #5 of the pyrimidine ring. This is the carbon that is substituted with a chemically active nitrogen in decitabine or 5-azacytidine, although in the case of TYMS, the substrate is deoxyuridine monophosphate (dUMP) instead of DNA-incorporated dCTP. A portion of administered decitabine, after phosphorylation by DCK then deamination by deoxycytidine deaminase (DCTD), is converted into a dUMP analog, Aza-dUMP. By depleting TYMS in this way, decitabine decreases dTTP that in turn increases dCTP, because dTTP inhibits ribonucleotide reductase-mediated reduction of CDP into dCDP. Decitabine inhibition of TYMS, and hence dTTP suppression/dCTP upregulation, has also been reported by others. A portion of administered 5-azacytidine can also be processed into Aza-dUMP, but via a more circuitous 6 instead of 2 catalytic steps. Instead, a more direct off-target action of 5-azacytidine, requiring only 2 catalytic steps to form Aza-CDP, is depletion of RRM1 - off-target inhibition of ribonucleotide reductase by 5-azacytidine, and hence dCTP suppression, has also been reported by others. In brief, differential effects of 5-azacytidine and decitabine on RRM1 vs TYMS drive dCTP levels in opposite directions, triggering distinct responses from the pyrimidine metabolism network: DCK is particularly important for preserving dCTP, shown by a decrease in dCTP in *DCK*-knockout cells (shown also by others). Hence, upregulation of DCK is an appropriate adaptive response to dCTP suppression by 5-azacytidine. Rapid upregulation of DCK by 5-azacytidine has also been observed by others¹⁵. UCK2 on the other hand appears particularly important for dTTP maintenance, shown by a decrease in dTTP in *UCK2*-knockout cells. Therefore, UCK2 upregulation is an appropriate response to dTTP suppression by decitabine. CDA also contributes to dTTP maintenance (additional references below), thus CDA upregulation is another appropriate response to dTTP suppression by decitabine, again also observed by others. Cytarabine, another deoxycytidine analog that inhibits TYMS, has also been found to rapidly upregulate CDA in vitro and in the clinic, while hydroxyurea that inhibits ribonucleotide reductase does not (additional references).

This mode of resistance, that emerges adaptively from metabolic networks purposed toward homeostasis, does not require genetic mutations, and consistent with this, several studies that have looked for correlations between MDS/AML mutations and decitabine/5-azacytidine resistance have generated inconclusive or contradictory results. Expression levels of pyrimidine metabolism enzymes at baseline may also not necessarily be predictive^{52, 53}, since the metabolic reconfigurations are molded by treatment_ENREF_60. We found that the consistent, predictable trajectory of the acute metabolic responses to the pro-drugs, however, facilitates outmaneuvering and even exploitation: (i) first, in PDX models of chemorefractory AML, scheduling decitabine administrations to avoid reactive troughs in DCK expression was notably superior to schedules that coincided with DCK troughs. (ii) Second, alternating decitabine with 5-azacytidine week-to-week, timed approximately to exploit priming of each pro-drug for activity of the other (UCK2 and DCK are maximally upregulated ~96 hrs after decitabine and 5-azacytidine respectively), was significantly superior to administration of either pro-drug alone. The timing of alternation was important - alternating the pro-drugs in 4 week cycles, or their simultaneous administration, did not add benefit over the single agents. (iii) Third, frequent, distributed pro-drug administration, to increase possibilities of overlap between malignant cell S-phase entries and drug exposure windows, was superior to pulse-cycled administration schedules. Pulse-cycled schedules concentrate treatment over a few consecutive days separated by multi-week intervals, recovery periods necessary with cytotoxic treatments - such long gaps are not needed if decitabine or 5-azacytidine doses are selected for non-cytotoxic DNMT1-depletion, as shown also in previous clinical trials. Observations from others support rationalization of treatment schedules to increase S-phase dependent DNMT1-depletion: RNA-sequencing analysis of patients' baseline bone marrows found that a gene expression signature of low cell cycle fraction predicted non-response to pulse-cycled 5-azacytidine therapy, and regulatory approval of decitabine and 5-azacytidine to treat myeloid malignancies occurred after doses were lowered from initially evaluated, toxic high doses, then administered more frequently¹. (iv) Fourth, adding THU, to inhibit the catabolic enzyme CDA that severely limits decitabine and 5-azacytidine tissue-distribution and half-lives, and that is rapidly upregulated by decitabine (and to a lesser extent 5-azacytidine) in vitro and in vivo, also extended decitabine or 5-azacytidine anti-AML, efficacy in vivo. An important detail in such combinations was that the decitabine and 5-azacytidine doses were lowered to preserve a non-cytotoxic DNMT1-targeting mode of action. Stated another way, dose-escalations of decitabine or 5-azacytidine are not a solution for resistance since this compromises therapeutic-index: AML cells indefinitely self-replicate/proliferate and therefore have the opportunity to be educated for resistance from repeated treatment exposures, but normal myelopoiesis proliferates and terminally differentiates in successive waves, each treatment naive and vulnerable to cytotoxic effects of high doses.

CAD, the enzyme that initiates de novo pyrimidine synthesis, was downregulated acutely by decitabine or 5-azacytidine, but was upregulated at stable resistance, the only discrepancy we found between acute vs chronic metabolic reconfiguration. This discrepancy may reflect the terminal-differentiation induced acutely but not at stable resistance. We did not find benefit in vivo from combining decitabine with dT or hydroxyurea to inhibit ribonucleotide reductase. Others, however, have found promise in vitro combining 5-azacytidine with other inhibitors of de novo pyrimidine synthesis: pyrazofurin to inhbit CTPS1⁵⁵, PALA to inhibit CAD¹⁷ or leflunomide to inhibit DHODH⁵⁶. As per combinations with CDA-inhibitors, 5-azacytidine or decitabine combinations with de novo synthesis inhibitors will likely require reductions in 5-azacytidine/decitabine doses to preserve therapeutic index, since toxicities caused failure of previous clinical trials of high dose 5-azacytidine and pyrazofurin⁵⁷.

In sum, we found that resistance to decitabine and 5-azacytidine emerges from adaptive responses of the pyrimidine metabolism network. These network responses can be anticipated and exploited using simple and practical treatment modifications that preserve the vital therapeutic index of non-cytotoxic DNMT1-depletion.

### REFERENCES

1. Saunthararajah Y. Key clinical observations after 5-azacytidine and decitabine treatment of myelodysplastic syndromes suggest practical solutions for better outcomes. Hematology Am Soc Hematol Educ Program 2013; 2013: 511-521.
2. Vesely J, Cihak A, Sorm F. Characteristics of mouse leukemic cells resistant to 5-azacytidine and 5-aza-2'-deoxycytidine. Cancer Res 1968 Oct; 28(10): 1995-2000.
3. Ng KP, Ebrahem Q, Negrotto S, Mahfouz RZ, Link KA, Hu Z, et al. p53 independent epigenetic-differentiation treatment in xenotransplant models of acute myeloid leukemia. Leukemia 2011 Nov; 25(11): 1739-1750.
4. Saunthararajah Y, Sekeres M, Advani A, Mahfouz R, Durkin L, Radivoyevitch T, et al. Evaluation of noncytotoxic DNMT1-depleting therapy in patients with myelodysplastic syndromes. J Clin Invest 2015 Mar 2; 125(3): 1043-1055.
5. Tsai HC, Li H, Van Neste L, Cai Y, Robert C, Rassool FV, et al. Transient Low Doses of DNA-Demethylating Agents Exert Durable Antitumor Effects on Hematological and Epithelial Tumor Cells. Cancer Cell 2012 Mar 20; 21(3): 430-446.
6. Negrotto S, Ng KP, Jankowska AM, Bodo J, Gopalan B, Guinta K, et al. CpG methylation patterns and decitabine treatment response in acute myeloid leukemia cells and normal hematopoietic precursors. Leukemia 2012 Feb; 26(2): 244-254.
7. Momparler RL, Cote S, Momparler LF. Epigenetic action of decitabine (5-aza-2'-deoxycytidine) is more effective against acute myeloid leukemia than cytotoxic action of cytarabine (ARA-C). Leuk Res 2013 May 6.
8. Trowbridge JJ, Sinha AU, Zhu N, Li M, Armstrong SA, Orkin SH. Haploinsufficiency of Dnmt1 impairs leukemia stem cell function through derepression of bivalent chromatin domains. Genes Dev 2012 Feb 15; 26(4): 344-349.
9. Milhem M, Mahmud N, Lavelle D, Araki H, DeSimone J, Saunthararajah Y, et al. Modification of hematopoietic stem cell fate by 5aza 2 ' deoxycytidine and trichostatin A. Blood 2004 Jun 1; 103(11): 4102-4110.
10. Hu Z, Negrotto S, Gu X, Mahfouz R, Ng KP, Ebrahem Q, et al. Decitabine maintains hematopoietic precursor self-renewal by preventing repression of stem cell genes by a differentiation-inducing stimulus. Mol Cancer Ther 2010 Jun; 9(6): 1536-1543.
11. Chaurasia P, Gajzer DC, Schaniel C, D'Souza S, Hoffman R. Epigenetic reprogramming induces the expansion of cord blood stem cells. J Clin Invest 2014 Apr 24.
12. Suzuki M, Harashima A, Okochi A, Yamamoto M, Nakamura S, Motoda R, et al. 5-Azacytidine supports the long-term repopulating activity of cord blood CD34(+) cells. AmJHematol 2004; 77(3): 313-315.
13. Velcheti V, Schrump D, Saunthararajah Y. Ultimate Precision: Targeting Cancer but Not Normal Self-replication. American Society of Clinical Oncology educational book American Society of Clinical Oncology Annual Meeting 2018 May 23; (38): 950-963.
14. Stegmann AP, Honders WH, Willemze R, Ruiz vHV, Landegent JE. Transfection of wild-type deoxycytidine kinase (dck) cDNA into an AraC- and DAC-resistant rat leukemic cell line of clonal origin fully restores drug sensitivity. Blood 1995; 85(5): 1188-1194.
15. Antonsson BE, Avramis VI, Nyce J, Holcenberg JS. Effect of 5-azacytidine and congeners on DNA methylation and expression of deoxycytidine kinase in the human lymphoid cell lines CCRF/CEM/0 and CCRF/CEM/dCk-1. Cancer Res 1987; 47(14): 3672-3678.
16. Qin T, Jelinek J, Si J, Shu J, Issa JP. Mechanisms of resistance to 5-aza-2'-deoxycytidine in human cancer cell lines. Blood 2009; 113(3): 659-667.
17. Grant S, Bhalla K, Gleyzer M. Effect of uridine on response of 5-azacytidine-resistant human leukemic cells to inhibitors of de novo pyrimidine synthesis. Cancer Res 1984 Dec; 44(12 Pt 1): 5505-5510.
18. Liacouras AS, Anderson EP. Uridine-cytidine kinase. IV. Kinetics of the competition between 5-azacytidine and the two natural substrates. Molecular pharmacology 1979 Mar; 15(2): 331-340.
19. Sripayap P, Nagai T, Uesawa M, Kobayashi H, Tsukahara T, Ohmine K, et al. Mechanisms of resistance to azacitidine in human leukemia cell lines. Exp Hematol 2014 Apr; 42(4): 294-306 e292.
20. Qin T, Castoro R, El Ahdab S, Jelinek J, Wang X, Si J, et al. Mechanisms of resistance to decitabine in the myelodysplastic syndrome. PLoS One 2011; 6(8): e23372.
21. Wu P, Geng S, Weng J, Deng C, Lu Z, Luo C, et al. The hENT1 and DCK genes underlie the decitabine response in patients with myelodysplastic syndrome. Leuk Res 2015 Feb; 39(2): 216-220.
22. Camiener GW, Smith CG. Studies of the enzymatic deamination of cytosine arabinoside. I. Enzyme distribution and species specificity. BiochemPharmacol 1965; 14(10): 1405-1416.
23. Zauri M, Berridge G, Thezenas ML, Pugh KM, Goldin R, Kessler BM, et al. CDA directs metabolism of epigenetic nucleosides revealing a therapeutic window in cancer. Nature 2015 Aug 6; 524(7563): 114-118.
24. Beausejour CM, Eliopoulos N, Momparler L, Le NL, Momparler RL. Selection of drug-resistant transduced cells with cytosine nucleoside analogs using the human cytidine deaminase gene. Cancer Gene Ther 2001; 8(9): 669-676.
25. Eliopoulos N, Cournoyer D, Momparler RL. Drug resistance to 5-aza-2'-deoxycytidine, 2',2'-difluorodeoxycytidine, and cytosine arabinoside conferred by retroviral-mediated transfer of human cytidine deaminase cDNA into murine cells. Cancer ChemotherPharmacol 1998; 42(5): 373-378.
26. Ebrahem Q, Mahfouz R, Ng KP, Saunthararajah Y. High cytidine deaminase expression in the liver provides sanctuary for cancer cells from decitabine treatment effects. Oncotarget 2012 Oct; 3(10): 1137-1145.
27. Liu Z, Marcucci G, Byrd JC, Grever M, Xiao J, Chan KK. Characterization of decomposition products and preclinical and low dose clinical pharmacokinetics of decitabine (5-aza-2'-deoxycytidine) by a new liquid chromatography/tandem mass spectrometry quantification method. Rapid CommunMass Spectrom 2006; 20(7): 1117-1126.
28. Mahfouz RZ, Jankowska A, Ebrahem Q, Gu X, Visconte V, Tabarroki A, et al. Increased CDA expression/activity in males contributes to decreased cytidine analog half-life and likely contributes to worse outcomes with 5-azacytidine or decitabine therapy. Clin Cancer Res 2013 Feb 15; 19(4): 938-948.
29. Mahfouz RZ, Koh LS, Teo M, Chee CL, Toh HC, Saunthararajah Y. Gender, cytidine deaminase, and 5-aza/decitabine--response. Clin Cancer Res 2013 Jun 1; 19(11): 3106-3107.
30. DeZern AE, Zeidan AM, Barnard J, Hand W, Al Ali N, Brown F, et al. Differential response to hypomethylating agents based on sex: a report on behalf of the MDS Clinical Research Consortium (MDS CRC). Leukemia & lymphoma 2017 Jun; 58(6): 1325-1331.
31. Grant S, Bhalla K, Gleyzer M. Interaction of deoxycytidine and deoxycytidine analogs in normal and leukemic human myeloid progenitor cells. LeukRes 1986; 10(9): 1139-1146.
32. Ng SK, Rogers J, Sanwal BD. Alterations in differentiation and pyrimidine pathway enzymes in 5-azacytidine resistant variants of a myoblast line. J Cell Physiol 1977 Feb; 90(2): 361-347.
33. Lane AN, Fan TW. Regulation of mammalian nucleotide metabolism and biosynthesis. Nucleic Acids Res 2015 Feb 27; 43(4): 2466-2485.
34. Almqvist H, Axelsson H, Jafari R, Dan C, Mateus A, Haraldsson M, et al. CETSA screening identifies known and novel thymidylate synthase inhibitors and slow intracellular activation of 5-fluorouracil. Nat Commun 2016; 7: 11040.
35. Heinemann V, Plunkett W. Modulation of deoxynucleotide metabolism by the deoxycytidylate deaminase inhibitor 3,4,5,6-tetrahydrodeoxyuridine. Biochemical pharmacology 1989 Nov 15; 38(22): 4115-4121.
36. Bianchi V, Pontis E, Reichard P. Regulation of pyrimidine deoxyribonucleotide metabolism by substrate cycles in dCMP deaminase-deficient V79 hamster cells. Mol Cell Biol 1987 Dec; 7(12): 4218-4224.
37. Nathanson DA, Armijo AL, Tom M, Li Z, Dimitrova E, Austin WR, et al. Co-targeting of convergent nucleotide biosynthetic pathways for leukemia eradication. J Exp Med 2014 Mar 10; 211(3): 473-486.
38. Gu X, Ebrahem Q, Mahfouz RZ, Hasipek M, Enane F, Radivoyevitch T, et al. Leukemogenic nucleophosmin mutation disrupts the transcription factor hub that regulates granulomonocytic fates. J Clin Invest 2018 Oct 1; 128(10): 4260-4279.
39. Saunthararajah Y, Hillery CA, Lavelle D, Molokie R, Dorn L, Bressler L, et al. Effects of 5-aza-2 '-deoxycytidine on fetal hemoglobin levels, red cell adhesion, and hematopoietic differentiation in patients with sickle cell disease. Blood 2003 Dec 1; 102(12): 3865-3870.
40. Molokie R, Lavelle D, Gowhari M, Pacini M, Krauz L, Hassan J, et al. Oral tetrahydrouridine and decitabine for non-cytotoxic epigenetic gene regulation in sickle cell disease: A randomized phase 1 study. PLoS medicine 2017 Sep; 14(9): e1002382.
41. Lavelle D, Vaitkus K, Ling Y, Ruiz MA, Mahfouz R, Ng KP, et al. Effects of tetrahydrouridine on pharmacokinetics and pharmacodynamics of oral decitabine. Blood 2012 Feb 2; 119(5): 1240-1247.
42. Aimiuwu J, Wang H, Chen P, Xie Z, Wang J, Liu S, et al. RNA-dependent inhibition of ribonucleotide reductase is a major pathway for 5-azacytidine activity in acute myeloid leukemia. Blood 2012 May 31; 119(22): 5229-5238.
43. Austin WR, Armijo AL, Campbell DO, Singh AS, Hsieh T, Nathanson D, et al. Nucleoside salvage pathway kinases regulate hematopoiesis by linking nucleotide metabolism with replication stress. J Exp Med 2012 Nov 19; 209(12): 2215-2228.
44. Momparler RL, Laliberte J. Induction of cytidine deaminase in HL-60 myeloid leukemic cells by 5-aza-2'-deoxycytidine. LeukRes 1990; 14(9): 751-754.
45. Mameri H, Bieche I, Meseure D, Marangoni E, Buhagiar-Labarchede G, Nicolas A, et al. Cytidine Deaminase Deficiency Reveals New Therapeutic Opportunities against Cancer. Clin Cancer Res 2017 Apr 15; 23(8): 2116-2126.
46. Im AP, Sehgal AR, Carroll MP, Smith BD, Tefferi A, Johnson DE, et al. DNMT3A and IDH mutations in acute myeloid leukemia and other myeloid malignancies: associations with prognosis and potential treatment strategies. Leukemia 2014 Sep; 28(9): 1774-1783.
47. DiNardo CD, Patel KP, Garcia-Manero G, Luthra R, Pierce S, Borthakur G, et al. Lack of association of IDH1, IDH2 and DNMT3A mutations with outcome in older patients with acute myeloid leukemia treated with hypomethylating agents. Leukemia & lymphoma 2014 Aug; 55(8): 1925-1929.
48. Traina F, Visconte V, Elson P, Tabarroki A, Jankowska AM, Hasrouni E, et al. Impact of molecular mutations on treatment response to DNMT inhibitors in myelodysplasia and related neoplasms. Leukemia 2014 Jan; 28(1): 78-87.
49. Metzeler KH, Walker A, Geyer S, Garzon R, Klisovic RB, Bloomfield CD, et al. DNMT3A mutations and response to the hypomethylating agent decitabine in acute myeloid leukemia. Leukemia 2012 May; 26(5): 1106-1107.
50. Bejar R, Lord A, Stevenson K, Bar-Natan M, Perez-Ladaga A, Zaneveld J, et al. TET2 mutations predict response to hypomethylating agents in myelodysplastic syndrome patients. Blood 2014 Oct 23; 124(17): 2705-2712.
51. Braun T, Itzykson R, Renneville A, de Renzis B, Dreyfus F, Laribi K, et al. Molecular predictors of response to decitabine in advanced chronic myelomonocytic leukemia: a phase 2 trial. Blood 2011 Oct 6; 118(14): 3824-3831.
52. Unnikrishnan A, Papaemmanuil E, Beck D, Deshpande NP, Verma A, Kumari A, et al. Integrative Genomics Identifies the Molecular Basis of Resistance to Azacitidine Therapy in Myelodysplastic Syndromes. Cell reports 2017 Jul 18; 20(3): 572-585.
53. Valencia A, Masala E, Rossi A, Martino A, Sanna A, Buchi F, et al. Expression of nucleoside-metabolizing enzymes in myelodysplastic syndromes and modulation of response to azacitidine. Leukemia 2014 Mar; 28(3): 621-628.
54. Awada H, Mahfouz RZ, Kishtagari A, Kuzmanovic T, Durrani J, Kerr CM, et al. Extended experience with a non-cytotoxic DNMT1-targeting regimen of decitabine to treat myeloid malignancies. Br J Haematol 2019 Nov 17.
55. Cadman E, Eiferman F, Heimer R, Davis L. Pyrazofurin enhancement of 5-azacytidine antitumor activity in L5178Y and human leukemia cells. Cancer Res 1978 Dec; 38(12): 4610-4617.
56. Imanishi S, Takahashi R, Katagiri S, Kobayashi C, Umezu T, Ohyashiki K, et al. Teriflunomide restores 5-azacytidine sensitivity via activation of pyrimidine salvage in 5-azacytidine-resistant leukemia cells. Oncotarget 2017 Sep 19; 8(41): 69906-69915.
57. Martelo OJ, Broun GO, Jr., Petruska PJ. Phase I study of pyrazofurin and 5-azacytidine in refractory adult acute leukemia. Cancer treatment reports 1981 Mar-Apr; 65(3-4): 237-239.
58. Wang H, Chen P, Wang J, Santhanam R, Aimiuwu J, Saradhi UV, et al. In vivo quantification of active decitabine-triphosphate metabolite: a novel pharmacoanalytical endpoint for optimization of hypomethylating therapy in acute myeloid leukemia. The AAPS journal 2013 Jan; 15(1): 242-249.
59. Gu X, Hu Z, Ebrahem Q, Crabb JS, Mahfouz RZ, Radivoyevitch T, et al. Runx1 regulation of Pu.1 corepressor/coactivator exchange identifies specific molecular targets for leukemia differentiation therapy. J Biol Chem 2014 May 23; 289(21): 14881-14895.
60. Hu Z, Gu X, Baraoidan K, Ibanez V, Sharma A, Kadkol S, et al. RUNX1 regulates corepressor interactions of PU.1. Blood 2011 Jun 16; 117(24): 6498-6508.

### Additional References:

1. de Saint Vincent BR, Dechamps M, Buttin G. The modulation of the thymidine triphosphate pool of Chinese hamster cells by dCMP deaminase and UDP reductase. Thymidine auxotrophy induced by CTP in dCMP deaminase-deficient lines. J Biol Chem 1980 Jan 10; 255(1): 162-167.
2. Moro-Bulnes A, Castillo-Acosta VM, Valente M, Carrero-Lerida J, Perez-Moreno G, Ruiz-Perez LM, et al. Contribution of Cytidine Deaminase to Thymidylate Biosynthesis in Trypanosoma brucei: Intracellular Localization and Properties of the Enzyme. mSphere 2019 Aug 7; 4(4).
3. Neuhard J. Pyrimidine nucleotide metabolism and pathways of thymidine triphosphate biosynthesis in Salmonella typhimurium. Journal of bacteriology 1968 Nov; 96(5): 1519-1527.
4. Andersen L, Kilstrup M, Neuhard J. Pyrimidine, purine and nitrogen control of cytosine deaminase synthesis in Escherichia coli K 12. Involvement of the glnLG and purR genes in the regulation of codA expression. Archives of microbiology 1989; 152(2): 115-118.
5. Momparler RL, Laliberte J. Induction of cytidine deaminase in HL-60 myeloid leukemic cells by 5-aza-2'-deoxycytidine. LeukRes 1990; 14(9): 751-754.
6. Mameri H, Bieche I, Meseure D, Marangoni E, Buhagiar-Labarchede G, Nicolas A, et al. Cytidine Deaminase Deficiency Reveals New Therapeutic Opportunities against Cancer. Clin Cancer Res 2017 Apr 15; 23(8): 2116-2126.
7. Meyers R, Malathi VG, Cox RP, Silber R. Studies on nucleoside deaminase. Increase in activity in HeLa cell cultures caused by cytosine arabinoside. J Biol Chem 1973 Sep 10; 248(17): 5909-5913.
8. Chen P, Aimiuwu J, Xie Z, Wei X, Liu S, Klisovic R, et al. Biochemical modulation of aracytidine (Ara-C) effects by GTI-2040, a ribonucleotide reductase inhibitor, in K562 human leukemia cells. The AAPS journal 2011 Mar; 13(1): 131-140.
9. Steuart CD, Burke PJ. Cytidine deaminase and the development of resistance to arabinosyl cytosine. NatNew Biol 1971; 233(38): 109-110.
10. Roberts D, Loehr EV. Depression of thymidylate synthetase activity in response to cytosine arabinoside. Cancer Res 1972 Jun; 32(6): 1160-1169.
11. International Patent Publication WO2017/096357.

## Claims

1. A cytidine deaminase inhibitor, decitabine and 5-azacytidine for use in a method of treating a patient with cancer via alternate administration comprising, or consisting essentially of:
treating said patient with cancer by alternate administration of decitabine and 5-azacytidine,
wherein the time between said administration of decitabine and 5-azacytidine is three to four days,
wherein said decitabine administration is combined with an inhibitor of the enzyme cytidine deaminase, and
wherein said 5-azacytidein administration is combined with an inhibitor of the enzyme cytidine deaminase.

2. The cytidine deaminase inhibitor, decitabine and 5-azacytidine for use in the method of Claim 1, wherein said patient does not receive any decitabine or 5-azacytidine between said administration of decitabine and 5-azacytidine.

3. The cytidine deaminase inhibitor, decitabine and 5-azacytidine for use in the method of Claim 1, wherein said treating by alternate administration is repeated at least five times or wherein said treating by alternate administration is repeated at least twelve times.

4. The cytidine deaminase inhibitor, decitabine and 5-azacytidine for use in the method of Claim 1, wherein said cancer is selected from the group consisting of: pancreatic cancer, a myeloid cancer, a lymphoid cancer, and small cell lung cancer.

5. The cytidine deaminase inhibitor, decitabine and 5-azacytidine for use in the method of Claim 1, wherein said patient is a human and said administering of said cytidine deaminase inhibitor is 15 mg/kg or 9-11 mg/kg of said cytidine deaminase inhibitor.

6. The cytidine deaminase inhibitor, decitabine and 5-azacytidine for use in the method of Claim 1, wherein said patient is a human and said administering of decitabine is 0.07-0.4 mg/kg of decitabine or 0.15-0.17 mg/kg of said decitabine.

7. The cytidine deaminase inhibitor, decitabine and 5-azacytidine for use in the method of Claim 1, further comprising: administering said patient a composition comprising human granulocyte colony-stimulating factor (GCSF).

8. The cytidine deaminase inhibitor, decitabine and 5-azacytidine for use in the method of Claim 1, further comprising: testing a sample from said patient to determine absolute neutrophil count (ANC), wherein said ANC is preferably below 1.5x10⁹, and said method further comprises administering said patient a composition comprising human granulocyte colony-stimulating factor (GCSF).

9. The cytidine deaminase inhibitor, decitabine and 5-azacytidine for use in the method of Claim 1, wherein said patient does not receive any cytidine deaminase inhibitor between said administration of decitabine and 5-azacytidine combined with said cytidine deaminase inhibitor.

10. The cytidine deaminase inhibitor, decitabine and 5-azacytidine for use in the method of Claim 1, wherein said cytidine deaminase inhibitor comprises tetrahydrouridine.

## Patentansprüche

1. Cytidindeaminase-Inhibitor, Decitabin und 5-Azacytidin zur Verwendung in einem Verfahren zur Behandlung eines Patienten mit Krebs mittels alternierender Verabreichung, umfassend oder im Wesentlichen bestehend aus:
Behandlung des Krebspatienten durch abwechselnde Verabreichung von Decitabin und 5-Azacytidin,
wobei der Zeitraum zwischen der Verabreichung von Decitabin und 5-Azacytidin drei bis vier Tage beträgt,
wobei die Verabreichung von Decitabin mit einem Inhibitor des Enzyms Cytidindeaminase kombiniert wird, und
wobei die Verabreichung von 5-Azacytidin mit einem Inhibitor des Enzyms Cytidindeaminase kombiniert wird.

2. Cytidindeaminase-Inhibitor, Decitabin und 5-Azacytidin zur Verwendung in dem Verfahren nach Anspruch 1, wobei der Patient zwischen der Verabreichung von Decitabin und 5-Azacytidin kein Decitabin oder 5-Azacytidin erhält.

3. Cytidindeaminase-Inhibitor, Decitabin und 5-Azacytidin zur Verwendung in dem Verfahren nach Anspruch 1, wobei die Behandlung durch abwechselnde Verabreichung mindestens fünfmal wiederholt wird oder wobei die Behandlung durch abwechselnde Verabreichung mindestens zwölfmal wiederholt wird.

4. Cytidindeaminase-Inhibitor, Decitabin und 5-Azacytidin zur Verwendung in dem Verfahren nach Anspruch 1, wobei der Krebs aus der Gruppe ausgewählt ist, die aus Bauchspeicheldrüsenkrebs, einem myeloischen Krebs, einem lymphatischen Krebs und kleinzelligem Lungenkrebs besteht.

5. Cytidindeaminase-Inhibitor, Decitabin und 5-Azacytidin zur Verwendung in dem Verfahren nach Anspruch 1, wobei der Patient ein Mensch ist und die Verabreichung des Cytidindeaminase-Inhibitors 15 mg/kg oder 9-11 mg/kg des Cytidindeaminase-Inhibitors beträgt.

6. Cytidindeaminase-Inhibitor, Decitabin und 5-Azacytidin zur Verwendung in dem Verfahren nach Anspruch 1, wobei der Patient ein Mensch ist und die Verabreichung von Decitabin 0,07-0,4 mg/kg Decitabin oder 0,15-0,17 mg/kg des Decitabins beträgt.

7. Cytidindeaminase-Inhibitor, Decitabin und 5-Azacytidin zur Verwendung in dem Verfahren nach Anspruch 1, umfassend ferner: Verabreichen einer Zusammensetzung, die humanen Granulozytenkolonie-stimulierenden Faktor (GCSF) umfasst, an den Patienten.

8. Cytidindeaminase-Inhibitor, Decitabin und 5-Azacytidin zur Verwendung in dem Verfahren nach Anspruch 1, umfassend ferner: Testen einer Probe von dem Patienten, um die absolute Neutrophilenzahl (ANC) zu bestimmen, wobei die ANC vorzugsweise unter 1,5 x 10⁹ liegt, und wobei das Verfahren ferner umfasst: Verabreichen einer Zusammensetzung, die humanen Granulozytenkolonie-stimulierenden Faktor (GCSF) umfasst, an den Patienten.

9. Cytidindeaminase-Inhibitor, Decitabin und 5-Azacytidin zur Verwendung in dem Verfahren nach Anspruch 1, wobei der Patient keinen Cytidindeaminase-Inhibitor erhält zwischen der Verabreichung von Decitabin und 5-Azacytidin in Kombination mit dem Cytidindeaminase-Inhibitor.

10. Cytidindeaminase-Inhibitor, Decitabin und 5-Azacytidin zur Verwendung in dem Verfahren nach Anspruch 1, wobei der Cytidindeaminase-Inhibitor Tetrahydrouridin umfasst.

## Revendications

1. Inhibiteur de cytidine désaminase, décitabine et 5-azacytidine pour utilisation dans un procédé de traitement d'un patient ayant un cancer par administration alternée comprenant, ou essentiellement constituée par :
un traitement dudit patient ayant un cancer par administration alternée de décitabine et de 5-azacytidine,
dans lequel le temps entre ladite administration de décitabine et de 5-azacytidine est de trois à quatre jours,
dans lequel ladite administration de décitabine est combinée avec un inhibiteur de l'enzyme cytidine désaminase, et
dans lequel ladite administration de 5-azacytidéine est combinée avec un inhibiteur de l'enzyme cytidine désaminase.

2. Inhibiteur de cytidine désaminase, décitabine et 5-azacytidine pour utilisation dans le procédé selon la revendication 1, dans lequel ledit patient ne reçoit pas de décitabine ni de 5-azacytidine entre ladite administration de décitabine et de 5-azacytidine.

3. Inhibiteur de cytidine désaminase, décitabine et 5-azacytidine pour utilisation dans le procédé selon la revendication 1, dans lequel ledit traitement par administration alternée est répété au moins cinq fois ou dans lequel ledit traitement par administration alternée est répété au moins douze fois.

4. Inhibiteur de cytidine désaminase, décitabine et 5-azacytidine pour utilisation dans le procédé selon la revendication 1, dans lequel ledit cancer est choisi dans le groupe constitué par : un cancer du pancréas, un cancer myéloïde, un cancer lymphoïde et un cancer du poumon à petites cellules.

5. Inhibiteur de cytidine désaminase, décitabine et 5-azacytidine pour utilisation dans le procédé selon la revendication 1, dans lequel ledit patient est un humain et ladite administration dudit inhibiteur de cytidine désaminase est de 15 mg/kg ou 9-11 mg/kg dudit inhibiteur de cytidine désaminase.

6. Inhibiteur de cytidine désaminase, décitabine et 5-azacytidine pour utilisation dans le procédé selon la revendication 1, dans lequel ledit patient est un humain et ladite administration de décitabine est de 0,07 à 0,4 mg/kg de décitabine ou de 0,15 à 0,17 mg/kg de ladite décitabine.

7. Inhibiteur de cytidine désaminase, décitabine et 5-azacytidine pour utilisation dans le procédé selon la revendication 1, comprenant en outre : l'administration audit patient d'une composition comprenant le facteur humain de stimulation des colonies de granulocytes (GCSF) .

8. Inhibiteur de cytidine désaminase, décitabine et 5-azacytidine pour utilisation dans le procédé selon la revendication 1, comprenant en outre : le test d'un échantillon dudit patient pour déterminer le nombre absolu de neutrophiles (ANC), dans lequel ledit ANC est de préférence inférieur à 1,5x10⁹, et ledit procédé comprend en outre l'administration audit patient d'une composition comprenant le facteur humain de stimulation des colonies de granulocytes (GCSF).

9. Inhibiteur de cytidine désaminase, décitabine et 5-azacytidine pour utilisation dans le procédé selon la revendication 1, dans lequel ledit patient ne reçoit aucun inhibiteur de cytidine désaminase entre ladite administration de décitabine et de 5-azacytidine combinée avec ledit inhibiteur de cytidine désaminase.

10. Inhibiteur de cytidine désaminase, décitabine et 5-azacytidine pour utilisation dans le procédé selon la revendication 1, dans lequel ledit inhibiteur de cytidine désaminase comprend de la tétrahydrouridine.
